# EUROPÄISCHE PATENTSCHRIFT

(11) **EP 2 696 924 B1**
(45) Veröffentlichungstag und Bekanntmachung des Hinweises auf die Patenterteilung: **31.08.2016**
(21) Anmeldenummer: 12715833.5
(22) Anmeldetag: 05.04.2012
(51) Int. Cl.: A61B 5/00, A61B 5/0205, A61B 5/021, A61B 5/04, A61B 5/0488, A61B 5/145, A61B 5/0402, A61M 16/00, H03H 17/02, H03H 21/00

(54) **VORRICHTUNG UND VERFAHREN ZUR DATENVERARBEITUNG PHYSIOLOGISCHER SIGNALE**
APPARATUS AND METHOD FOR DATA PROCESSING FOR PHYSIOLOGICAL SIGNALS
DISPOSITIF ET PROCÉDÉ DE TRAITEMENT DE DONNÉES DE SIGNAUX PHYSIOLOGIQUES

(30) Priorität: 12.04.2011 DE 102011016804
(43) Veröffentlichungstag der Anmeldung: 19.02.2014
(73) Patentinhaber: Drägerwerk AG & Co. KGaA, 23558 Lübeck (DE)
(72) Erfinder: SATTLER, Frank, 23560 Lübeck (DE); EGER, Marcus, 23562 Lübeck (DE)
(74) Vertreter: Uexküll & Stolberg
(86) Internationale Anmeldenummer: PCT/EP2012/001523
(87) Internationale Veröffentlichungsnummer: WO 2012/139737

(56) Entgegenhaltungen:
- US-A- 5 259 387
- US-A1- 2006 056 641
- US-A1- 2008 288 199
- US-A1- 2008 308 104
- US-A1- 2009 122 930

## Beschreibung

Die vorliegende Erfindung betrifft allgemein das Gebiet der Verarbeitung physiologischer Signale und insbesondere eine Vorrichtung und ein Verfahren zur Verarbeitung physiologischer Signale, speziell physiologische Signale von Menschen, für therapeutische und diagnostische Anwendungen sowie für Überwachungsanwendungen ("Monitoring"). Ein Spezialfall ist die Anwendung einer solchen Signal- bzw. Datenverarbeitung unter Verwendung einer Filtervorrichtung bei der Steuerung der Beatmung eines Patienten durch ein Beatmungsgerät

Typischerweise können Signale von Sensoren, die der Erfassung physiologischer Eigenschaften dienen, nicht direkt weiterverwendet werden, sondern müssen geeignet gefiltert werden. Dies dient insbesondere dem Zweck, das gewünschte Nutzsignal, zum Beispiel ein EKG-Signal, von unerwünschten Signalen, etwa Rauschen oder Bewegungsartefakten, zu trennen, um dadurch das eigentliche Nutzsignal deutlicher hervortreten zu lassen.

Zur Durchführung einer solchen Filterung sind seit langem verschiedene Verfahren bekannt. Diese Verfahren basieren zumeist auf sogenannten linearen Filtern, deren Beschreibung durch lineare mathematische Transformationen (z.B. Fouriertransformation) erfolgen kann. Lineare Filter lassen sich vergleichsweise einfach entwerfen und besitzen signalunabhängige Eigenschaften, d.h. die Eigenschaften hängen nicht vom verarbeiteten Signal ab. Typischerweise sind solche Filter durch eine sogenannte Systemfunktion charakterisiert, die den Frequenzgang und den Phasengang widerspiegelt.

Grundsätzlich ist jedoch jede Form der Signalverarbeitung ("Filterung") mit einem gewissen Zeitaufwand verbunden. Dies bedeutet, dass das verarbeitete Signal erst nach einer bestimmten Verzögerungszeit am Ausgang der Signalverarbeitungseinheit zur Verfügung steht. Die Größe der Verzögerungszeit hängt dabei ganz entscheidend von den Filtereigenschaften ab.

Bei einigen Anwendungen, wie insbesondere bei der Verarbeitung physiologischer Signale, darf diese Verzögerungszeit jedoch eine bestimmte Grenze nicht überschreiten, da z.B. ein medizintechnisches Gerät sein Verhalten schnell genug ändern muss, um seinen Zweck zu erfüllen. Beispielsweise muss ein Beatmungsgerät schnell genug auf den Beatmungswunsch eines Patienten reagieren können, da eine Beatmung sonst nicht adäquat durchgeführt werden kann.

Da es physiologische Signale gibt, die einerseits einen hohen Filteraufwand, d.h. eine hohe Filterqualität, benötigen und andererseits aber nur geringe Verzögerungszeiten zulässig sind, entsteht hier eine besonders hohe Anforderung an die Signalverarbeitung, d.h. an das verwendete Filter.

Lineare Filter kommen hierbei schnell an ihre Grenzen, da insbesondere deren Fähigkeit Signale zu trennen ("Filtersteilheit" bzw. "Bandbreite") direkt mit der Verzögerungszeit zusammenhängt. Genau genommen gibt es einen Zusammenhang zwischen der Einschwingzeit und der dazu benötigten Frequenzbandbreite. In der Theorie der Filter spricht man hier vom Zeit-Bandbreite-Produkt. Dieses besagt, dass das Produkt aus Einschwingzeit und Frequenzbandbreite konstant ist. Dieser Zusammenhang ist fundamental und liegt an den linearen Eigenschaften dieser Filter. Es gibt deshalb keine Möglichkeit, schmalbandige bzw. steilflankige Filter mit sehr kleiner Verzögerungszeit zu bauen. Lange Einschwingzeiten bedeuten hierbei, dass das Filter Artefakte erzeugt, die von langer Dauer sind. Mit anderen Worten, jede kurze Änderung im Eingangssignal erzeugt am Ausgang einen Filterartefakt mit langer Dauer. Dieses Phänomen ist unter dem Begriff "Einschwingverhalten" bekannt Anders und vereinfacht gesagt zeigen lineare Filter entweder schlechte Eigenschaften bei kurzen Verzögerungszeiten oder gute Eigenschaften bei langen Verzögerungszeiten.

Neben den klassischen linearen Filtern gibt es Filter, die im Gegensatz zu linearen Filtern zum Teil zeitvariante Signale verarbeiten können und zum Teil nicht auf linearen Verfahren basieren. Beispiele hierfür sind Blind Source Separation, Independent Component Analysis, Principal Component Analysis, Adaptive Filter und die Empirical Mode Decomposition. Obwohl diese Filter zum Teil erheblich bessere Eigenschaften als klassische lineare Filter haben, ist der damit verbundene Signalverarbeitungsaufwand hoch, und immer ergeben sich mehr oder weniger große Verzögerungszeiten. Deshalb sind diese Filter nicht für alle Anwendungen nutzbar.

Für diese Anwendungen kommen deshalb nur andersartige nichtlineare Filter in Betracht. Insbesondere lassen sich bei nichtlinearen Filtern die Einschwingzeit bzw. die Dauer von Filterartefakten und die Filterqualität ("Filtersteilheit" bzw. "Bandbreite") voneinander entkoppeln. Ein Beispiel für ein solches Filter stellt ein Schwellwertdetektor dar, der alle Werte oberhalb einer bestimmten Schwelle z.B. auf Null setzt. Hier ist offensichtlich die Verzögerungszeit sehr klein, da ein Schwellwertvergleich sehr schnell durchführbar ist und ferner keine Filterartefakte auftreten. Folglich gibt es bei diesen Filtern kein Nachschwingen.

Aus dem Stand der Technik sind verschiedene Filterlösungen bekannt.

So beschreibt die DE 42 35 318 C2 eine Vorrichtung zum Entfernen einer Grundlinienschwankung von einem EKG-Signal, bei der ein Vorwärtsfilter und ein Rückwärtsfilter über einen Puffer seriell verbunden sind. Hierbei wird ein EKG-Signal an das Vorwärtsfilter angelegt, das ein nichtlineares Phasenantwortverhalten hat und gefilterte Signaldaten erzeugt. Diese Daten werden in dem Puffer gespeichert, und Blöcke der gespeicherten Daten werden in zeitlich umgekehrter Reihenfolge an das ebenfalls nichtlineare Rückwärtsfilter angelegt, um auf diese Weise in Rückwärtsrichtung gefilterte Blöcke zu erzeugen.

Die DE 197 28 782 B4 betrifft ein nichtlineares Filter für schwingungsbehaftete Gebersignale zum Einsatz in Kraftfahrzeugen. Die Gebersignale werden an einen Eingang des Filters angelegt, wobei in einem Differenzbildungsglied eine Differenz eines Ausgangssignals des Filters von diesem Eingangssignal gebildet wird und die so gebildete Differenz einmal direkt und einmal über ein nichtlineares Übertragungsglied einem Integrierglied aufgeschaltet wird. Das nichtlineare Übertragungsglied ist mit einer in ihrer Breite veränderlichen Mittelzone relativ geringer Verstärkung ausgestattet, wobei die Breite der Mittelzone durch einen die Amplitudendifferenz zwischen dem größten und kleinsten Signalwert des Filtereingangssignals während jeder Schwingungsperiodendauer der störenden Schwingung im Eingangssignal bildenden Amplitudendetektor so nachgeführt wird, dass die Zeitkonstante des Filters nur während Änderungen des Nutzsignals auf einen kleinen Wert verringert wird und deshalb das Störsignal am Filterausgang nicht erscheint.

Die DE 195 18 528 A1 offenbart ein digitales Hochpassfilter mit Einrichtungen zur Wiederherstellung der Grundlinien, wobei das Hochpassfilter von einem Tiefpassfilter abgeleitet ist. Das Tiefpassfilter wird durch erste und zweite Tiefpassfilter gebildet, von denen jeder ein gemeinsames Eingangssignal empfängt und ein zugehöriges Ausgangssignal ausgibt. Hierbei hat das erste Filter eine relativ hohe Grenzfrequenz, so dass es genau dem Eingangssignal folgt, jedoch nicht dessen Welligkeit signifikant dämpft. Das zweite Filter hat eine Grenzfrequenz, die in Abhängigkeit von einem Steuersignal geändert werden kann, welches auf Basis eines Vergleichs der Ausgangssignale beider Filter erzeugt wird.

Die EP 0 677 922 A2 betrifft allgemein ein schnelles Tiefpassfilter und ein langsames Tiefpassfilter, wobei beide Filter das gleiche Eingangssignal empfangen und die Ausgaben beider Filter verglichen werden, um die Antwortzeit des langsamen Tiefpassfilter zu erhöhen, wenn die Differenz der Ausgaben einen bestimmten Wert übersteigt, so dass das langsame Tiefpassfilter dem Eingangssignal schneller folgen kann.

Die EP 0 749 056 B1 zeigt ein rückgekoppeltes Nachlauffilter unter Verwendung mehrerer Integratoren.

Die EP 1 346 743 A1 offenbart eine Vorrichtung zur Steuerung eines Beatmungsgeräts mit einer Abgabesteuerung für Atemgas, die an einen Sensor für ein Messsignal angeschlossen ist und einen Druckaufbau in Abhängigkeit von einem zum Messsignal korrespondierenden Triggersignal durchführt.

Die EP 1 793 374 A1 betrifft eine Filtervorrichtung zur aktiven Rauschreduktion.

Die US 4,248,240 zeigt eine Vorrichtung zum Detektieren der Aktivität der Atmungsorgane und des Herzens von Lebewesen, bei der EMG-Signale mit Hilfe eines Hochpassfilters gefiltert werden, dem ein Komparator parallel geschaltet ist.

Die US 5,777,909 betrifft ein Tiefpassfilter mit Koeffizientenumschaltung zur Verbesserung der Einschwingzeit.

Die US 7,535,859 B2 beschäftigt sich mit dem Problem der Sprachaktivitätserkennung unter Verwendung adaptiver Verfolgung des Grundrauschens.

Die US 2009/0143693 A1 beschreibt allgemein eine Vorrichtung zur Erzeugung von Bestimmungsindizes, um EKG-Störsignale zu identifizieren.

Die US 2010/0168595 A1 betrifft ein Verfahren und eine Vorrichtung zur Beseitigung einer Grundlinienverschiebung mit einem ersten und einem zweiten Verstärker, dem ein Tiefpassfilter zwischengeschaltet ist. Die Ausgabe des ersten Verstärkers wird dem Tiefpassfilter als auch einer dem Tiefpassfilter parallel geschalteten Zeitverzögerungsschaltung zugeführt, deren Ausgangssignal zum Ausgangssignal des Tiefpassfilters addiert und dem zweiten Verstärker zugeführt wird.

Die US 2008/0288199 A1 offenbart ein Verfahren zur Signalverarbeitung, mit Empfangen eines gestörten Signals, das eine gestörte Komponente und eine ungestörte Komponente enthält, wobei die gestörte Komponente zumindest teilweise mit einem exogenen Signal in Beziehung steht, und Durchführen einer Linearisierung, die zumindest teilweise auf dem gestörten Signal und auf Informationen basiert, die mit dem exogenen Signal in Beziehung stehen, um ein korrigiertes Signal zu erhalten, das der ungestörten Komponente im Wesentlichen ähnlich ist. Eine adaptives System zum Reduzieren von Störungen umfasst eine Eingangsschnittstelle, die konfiguriert ist, um ein gestörtes Signal zu empfangen, das eine gestörte Komponente und eine ungestörte Komponente enthält, wobei die gestörte Komponente mindestens teilweise mit einem exogenen Signal in Beziehung steht, und ein adaptives Modul zum Reduzieren von Störungen, das mit der Eingangsschnittstelle gekoppelt ist, ist konfiguriert, um die Linearisierung durchzuführen, die zumindest teilweise auf dem gestörten Signal und auf Informationen basiert, die mit dem exogenen Signal in Beziehung stehen, um ein korrigiertes Signal zu erhalten, das der ungestörten Komponente im Wesentlichen ähnlich ist.

Die US 5,259,387 beschreibt ein Filtersystem zum Entfernen von hochfrequenten Signalen mit kleiner Amplitude, wie Muskel-Artefakt-Signale, aus einem EKG-Signal. Das Filtersystem umfasst ein Tiefpassfilter mit variablen Grenzfrequenzen, eine elektronische Verzögerung und ein System zum Erfassen der R-Zacke in dem EKG-Signal und zum Bestimmen der variablen Grenzfrequenz in Reaktion auf die Erfassung der R-Zacke. Ein digitalisiertes EKG-Eingangssignal wird gleichzeitig an die elektronische Verzögerung und an das System zur Erfassung der R-Zacke geliefert. Während des Bereichs des EKG-Signals, der den QRS-Komplex nicht enthält, wird der Filter mit einer niedrigen Grenzfrequenz betrieben, um die Muskel-Artefakt-Signale mit maximaler Glättung zu filtern. Zu einem Zeitpunkt kurz vor dem QRS-Komplex wird die Grenzfrequenz schnell und schrittweise auf eine höhere Grenzfrequenz erhöht, um den QRS-Komplex mit einer minimalen Verringerung der Amplitude des QRS-Signals durchzulassen. Am Ende des QRS-Signals wird die Grenzfrequenz des Filters schnell und schrittweise auf die niedrige Grenzfrequenz zurückgesetzt.

Die US 2009/0122930 A1 betrifft eine Vorrichtung mit einer Filtereinheit, die eine Mehrzahl von verschiedenen Filtern aufweist, die jeweils konfiguriert sind, um ein erstes Signal zu filtern, wenn es ausgewählt ist. Die Vorrichtung kann ferner eine Steuereinheit aufweisen, die konfiguriert ist, um eines der Mehrzahl der Filter in Abhängigkeit von einer Stärke des ersten Signals auszuwählen.

Als weiterer Stand der Technik sei genannt: AU 707148 B2; DE 101 64 446 A1; DE 10 2007 024 072 A1; DE 10 2007 062 214 B3; EP 0 889 291 A2; EP 1 365 296 A1; US 4,915,103; US 5,353,788; US 5,980 463; US 6,588,423; US 2004/0260186 A1; US 2006/0152197 A1; WO 98/48877; WO 2006/131149 A1; WO 2006/029529 A1; DE 199 59 822 A1; US 5,820,560 A und WO 2009/096820 A1.

Die Aufgabe der vorliegenden Erfindung besteht allgemein in der Überwindung der Nachteile des Standes der Technik und insbesondere in der Realisierung einer schnellen Echtzeitfilterung bei gleichzeitiger Realisierung einer hohen Filterqualität, speziell im Bereich medizinischer Geräte, wie zum Beispiel Beatmungsgeräte.

Die Aufgabe wird gelöst durch eine Vorrichtung mit den Merkmalen des Anspruchs 1.

Die Aufgabe wird ebenfalls gelöst durch eine Vorrichtung mit den Merkmalen des Anspruchs 3.

Die Aufgabe wird ebenfalls gelöst durch ein Verfahren mit den Merkmalen des Anspruchs 5.

Geeignete Ausführungsformen ergeben sich aus Unteranspruch 2 bzw. 4 sowie aus den Unteransprüchen 6 und 7.

Gemäß einem bevorzugten Ausführungsbeispiel der vorliegenden Erfindung ist eine Filtervorrichtung zur Verwendung bei der Steuerung eines Beatmungsgeräts vorgesehen, mit:
einem Signaleingang, an dem ein Eingangssignal anliegt, das einen Nutzanteil und einen Störanteil enthält;
einem schnellen Signalpfad und einem parallel dazu angeordneten langsamen Signalpfad;
wobei der Signaleingang mit Eingängen des schnellen Signalpfades und des langsamen Signalpfades gekoppelt ist;
wobei der schnelle Signalpfad ein Filter enthält, um eine schnelle Filterung des Eingangssignals zu bewirken;
wobei der langsame Signalpfad ein Filter enthält, um eine langsame Filterung des Eingangssignals zu bewirken;
wobei die Signaldurchlaufzeit im schnellen Signalpfad mindestens um den Faktor 2 geringer ist als die Signaldurchlaufzeit im langsamen Signalpfad;
wobei ein Ausgang des langsamen Signalpfades über eine Signalleitung mit dem schnellen Signalpfad gekoppelt ist;
einem Signalausgang, der mit einem Ausgang des schnellen Signalpfades gekoppelt ist und an dem ein Ausgangssignal anliegt, das im Wesentlichen Nutzanteile des Eingangssignals enthält; und
einer weiteren Signalleitung, über die ein Signal von dem schnellen Signalpfad an den langsamen Signalpfad zurückgeführt wird, um das funktionale Verhalten des langsamen Signalpfades zu beeinflussen;
wobei ein Ausgang des schnellen Signalpfades über eine Signalleitung mit einem Eingang des langsamen Signalpfades gekoppelt ist; und
wobei an dem Signaleingang physiologische Signale von einem Patienten anliegen, die nach ihrer Filterung zur Ansteuerung eines Beatmungsgerätes verwendet werden.

Gemäß einem weiteren bevorzugten Ausführungsbeispiel der Erfindung ist eine Filtervorrichtung zur Verwendung bei der Steuerung eines Beatmungsgeräts vorgesehen, mit
einer Mehrzahl von Signaleingängen, an denen jeweils ein Eingangssignal anliegt, das einen Nutzanteil und einen Störanteil enthält;
einer Mehrzahl von parallel angeordneten schnellen Signalpfaden;
einer Mehrzahl von parallel angeordneten langsamen Signalpfaden;
wobei jeder der Mehrzahl von Signaleingängen jeweils mit einem Eingang der Mehrzahl von parallel angeordneten schnellen Signalpfaden und der Mehrzahl von parallel angeordneten langsamen Signalpfaden gekoppelt ist;
wobei jeder schnelle Signalpfad ein Filter enthält, um eine schnelle Filterung des Eingangssignals zu bewirken;
wobei jeder langsame Signalpfad ein Filter enthält, um eine langsame Filterung des Eingangssignals zu bewirken;
wobei die Signaldurchlaufzeit im schnellen Signalpfad mindestens um den Faktor 2 geringer ist als die Signaldurchlaufzeit im langsamen Signalpfad;
wobei jeder langsame Signalpfad über jeweilige Signalleitungen mit zugehörigen Eingängen einer Verrechnungs-/Entscheidungseinheit gekoppelt ist;
wobei jeder schnelle Signalpfad über jeweilige Signalleitungen mit zugehörigen Ausgängen der Verrechnungs-/Entscheidungseinheit gekoppelt ist; und
einer Mehrzahl von Signalausgängen, die jeweils mit Ausgängen der schnellen Signalpfade gekoppelt sind und an denen jeweils Ausgangssignale anliegen, die im Wesentlichen Nutzanteile der Eingangssignale enthalten.

Die vorliegende Erfindung betrifft außerdem ein Verfahren zur Echtzeitfilterung physiologischer Signale zur Verwendung bei der Steuerung eines Beatmungsgeräts
mit einem Nutzanteil und mit einem Störanteil, umfassend einen physiologischen Signal zu durchlaufenden schnellen Signalpfad mit einem Signaleingang und einem Signalausgang und umfassend einen parallel dazu vom physiologischen Signal zu durchlaufenden langsamen Signalpfad mit einem Signaleingang und einem Signalausgang, umfassend eine Verbindung, welche den Signalausgang des schnellen Signalpfades mit dem Signalausgang des langsamen Signalpfades als einen Signalausgang verbindet und umfassend eine weitere Verbindung, welche den Signalausgang des schnellen Signalpfades mit dem Signaleingang des langsamen Signalpfades verbindet,
wobei das physiologische Signal parallel in den Eingang des schnellen Signalpfades und in den Eingang des langsamen Signalpfades eingekoppelt wird, wobei der schnelle Signalpfad schnell durchlaufen wird und am Signalausgang des schnellen Signalpfades ein gefiltertes Ausgangssignal anliegt, das im Wesentlichen den Nutzanteil des physiologischen Signals enthält und in dem der Störanteil des physiologischen Signals nicht vollständig durch die schnelle Filterung unterdrückt ist, wobei über die Verbindung das Verhalten des schnellen Signalpfades beeinflusst wird, wobei der langsame Signalpfad langsam durchlaufen wird, wobei über die weitere Verbindung das Verhalten des langsamen Signalpfades beeinflusst wird und wobei am Signalausgang des langsamen Signalpfades ein gefiltertes Ausgangssignal anliegt, das im Wesentlichen den Nutzanteil des physiologischen Signals enthält und in dem der Störanteil des physiologischen Signals im Wesentlichen durch die langsame Filterung unterdrückt ist, wobei am Signalausgang unverzögert gegenüber dem Signalausgang des schnellen Signalpfades ein gefiltertes Ausgangssignal anliegt, das im Wesentlichen den Nutzanteil des physiologischen Signals enthält und in dem der Störanteil des physiologischen Signals im Wesentlichen unterdrückt ist, und wobei die Signaldurchlaufzeit im schnellen Signalpfad mindestens um den Faktor 2 geringer ist als die Signaldurchlaufzeit im langsamen Signalpfad.

Die vorliegende Erfindung betrifft ferner ein Verfahren zur Echtzeitfilterung physiologischer Signale mit einem Nutzanteil und einem Störanteil zur Verwendung bei der Steuerung eines Beatmungsgeräts, wobei
an einem Signaleingang eines schnellen Signalpfades und parallel und zeitlich gleichzeitig dazu in einen Signaleingang eines langsamen Signalpfad ein gestörtes physiologisches Signal als Eingangssignal eingespeist wird,
im schnellen Signalpfad eine schnelle Filterung durchlaufen wird,
im langsamen Signalpfad eine langsame Filterung durchlaufen wird,
die Signaldurchlaufzeit im schnellen Signalpfad mindestens um den Faktor 2 geringer ist als die Signaldurchlaufzeit im langsamen Signalpfad,
ein Parameter des langsamen Signalpfades in den schnellen Signalpfad eingespeist wird,
ein Rückführungssignal des schnellen Signalpfades in den langsamen Signalpfad zurück geführt wird,
ein entstörtes physiologisches Signal als gefiltertes Ausgangssignal anliegt, welches gegenüber dem Durchlauf des schnellen Signalpfades unverzögert ist.

Die Erfindung wird anhand von Ausführungsbeispielen und Figuren im Folgenden näher erläutert.

Im Sinne der vorliegenden Erfindung wird der Begriff Echtzeitfilterung in dem Sinne verstanden, dass eine Filterung des Signals auf aktuell erfasste Messwerte angewendet wird und die Ergebnisse ohne wesentlichen zeitlichen Verzug, d.h. ohne oder mit geringer Verzögerung oder mit vernachlässigbar kleiner Verzögerung zur Verfügung stehen.

Unter einer geringen Verzögerung ist im Sinne der vorliegenden Erfindung eine Verzögerung eines Signals zu verstehen, die bei einer an die Signalbandbreite angepassten Abtastung und Auslegung der analogen Schaltungskomponenten durch analoge Signalverarbeitung und Verstärkung, Analog- zu Digitalwandlung, mathematische Umrechnungen und Normierungen, Digital- zu Analogwandlung, keinerlei Auswirkungen und Informationsverlust hinsichtlich einer unverfälschten Wiedergabe oder Weiterverarbeitung des Signals zur Folge haben. Als ein orientierendes Maß für eine an die Signalbandbreite angepasste Abtastung ist das Nyquist- Kriterium zu Grunde zu legen.

Die in der vorliegenden Erfindung gewählten Ausführungsbeispiele beschreiben die Filterung anhand von mindestens zwei parallelen Signalpfaden, an die zeitlich gleichzeitig ein gemeinsames Eingangssignal angelegt wird. Der Begriff "parallele Signalpfade" ist jedoch nicht auf eine zwingende Parallelität im Sinne von Multi-Processing beschränkt, sondern beschreibt vielmehr eine "funktionale" Parallelität. Im Sinne der vorliegenden Erfindung ist als "funktionale" Parallelität beispielsweise eine Verarbeitung von Signalen in gleichsam zeitgleicher Weise zu verstehen, z.B. in einem Multiplexing- oder Multitasking-Verfahren, wobei die Verarbeitung auch mittels lediglich einer arithmetisch-logischen Recheneinheit (ALU), einem µC, µP oder ähnlichem Prozessortyp erfolgen kann. Jeder der mindestens zwei Signalpfade wird vom Eingangssignal durchlaufen und dabei spezifisch in jedem dieser mindestens zwei Signalpfade gefiltert. Die Ausgangssignale dieser mindestens zwei Signalpfade werden miteinander in einer Weise verbunden, dass mindestens einer der mindestens zwei Signalpfade den anderen der mindestens zwei Signalpfade beeinflusst und somit auf dessen Ausgangssignal einwirkt, sodass am Ausgang mindestens einen Signalpfades durch die Einwirkung des mindestens einen anderen Signalpfades eine Verbesserung der Signalfilterung erreicht wird.

Eine Signalfilterung und eine Echtzeitfilterung umfassen im Sinne der vorliegenden Erfindung verschiedenste Arten der Filterung, welche im Ergebnis im Sinne der vorliegenden Erfindung gleichwirkend sind. So sind sowohl Filterungen basierend auf elektronischen Bauteilen, wie beispielsweise basierend auf Operationsverstärker-Schaltungen mit weiteren Bauteilen, wie Dioden, Widerständen, Kondensatoren oder Spulen, auch in geeigneter Kombination mit Spitzenwertdetektoren, Gleichrichtern, Schieberegistern und anderen Logikschaltungen im Sinne der vorliegenden Erfindung umfasst. Eine beispielhafte Schaltungsvariante mit Operationsverstärkern ist als Sallen-Key- Schaltung dem Fachmann bekannt. Weiterhin sind Filterschaltungen basierend auf programmierbaren Bauteilen im Sinne der vorliegenden Erfindung mit umfasst. Als nicht abschließende Auflistung programmierbarer Typen von Bauteilen sind hier beispielhaft FPGA (Field Programmable Gate Array), PAL (Programmable Array Logic), GAL (Generic Array Logic), ASIC (Application-specific integrated circuit), CPLD (Complex Programmable Logic Device) oder weitere analoge programmierbare Bausteine, digitale programmierbare Bausteine, sowie Kombinationen der in der Auflistung genannten Typen genannt.

Weiterhin sind auch verschiedene Arten digitaler Filterungen, welche nach einer Analogzu- Digitalwandlung in Form einer in Software implementierten Filterung durch eine Ablaufsteuerung in Form eines programmierten Quellcodes, in denen Filterkoeffizienten und / oder mathematische Gleichungsmodelle enthalten sind, welche mit Hilfe eines µP's (Micro Processor), µC's (Micro Controller) oder eines DSP's (Digital Signal Processor) in verschiedenen Ausführungen von Filtertypen ausgeführt werden, im Sinne der vorliegenden Erfindung als Möglichkeiten einer Signalfilterung und einer Echtzeitfilterung mit umfasst. Zu solchen, auf programmierbarem Quellcode basierenden Filtertypen gehören beispielsweise FIR- Filter (Finite Impulse Response), IIR- Filter (Infinite Impulse Response), aber auch Glättungsfilter, Kalman- Filter, nichtlineare Filter wie Begrenzungsfilter oder Rangordnungsfilter.

Eine Signalfilterung und eine Echtzeitfilterung im Sinne der vorliegenden Erfindung findet beispielsweise Anwendung bei der Ansteuerung eines Beatmungsgeräts auf Basis entsprechend gefilterter physiologischer Signale eines Patienten. Eine solche Beatmungssteuerung erfordert eine minimale Verzögerungszeit bei der Filterung des physiologischen Signals, so dass die Beatmungssteuerung ohne wesentliche zeitliche Verzögerung auf sich ändernde Beatmungsanforderungen reagieren kann.

Das nachfolgend beschriebene erfindungsgemäße Verfahren zur Echtzeitfilterung physiologischer Signale ermöglicht eine Trennung des Nutzanteiles eines physiologischen Signals von Störanteilen.

Das Verfahren zur Echtzeitfilterung physiologischer Signale mit einem Nutzanteil und mit einem Störanteil, umfasst einen schnellen Signalpfad und einen parallelen langsamen Signalpfad, welche vom physiologischen Signal durchlaufen werden,
mit einer Verbindung zwischen den Signalausgängen des schnellen Signalpfades und des langsamen Signalpfades und einer weiteren Verbindung zwischen dem Signalausgang des schnellen Signalpfades mit dem Signaleingang des langsamen Signalpfades,
wobei das physiologische Signal parallel in den Eingang des schnellen Signalpfades und in den Eingang des langsamen Signalpfad eingekoppelt wird,
wobei der schnelle Signalpfad schnell durchlaufen wird und eine schnelle, vorzugsweise nichtlineare Filterung vorgenommen wird und am Signalausgang des schnellen Signalpfades ein gefiltertes Ausgangssignal anliegt, das im Wesentlichen den Nutzanteil des physiologischen Signals enthält und in dem der Störanteil des physiologischen Signals nicht vollständig durch die schnelle Filterung unterdrückt ist,
wobei über die Verbindung das Verhalten des schnellen Signalpfades beeinflusst wird,
wobei der langsame Signalpfad langsam durchlaufen wird und eine langsame, vorzugsweise nichtlineare Filterung vorgenommen wird,
wobei über die weitere Verbindung das Verhalten des langsamen Signalpfades beeinflusst wird
und wobei am Signalausgang des langsamen Signalpfades ein gefiltertes Ausgangssignal anliegt, das im Wesentlichen den Nutzanteil des physiologischen Signals enthält und in dem der Störanteil des physiologischen Signals im Wesentlichen durch die langsame Filterung unterdrückt ist,
wobei am Signalausgang unverzögert gegenüber dem Signalausgang des schnellen Signalpfades ein gefiltertes Ausgangssignal anliegt, das im Wesentlichen den Nutzanteil des physiologischen Signals enthält und in dem der Störanteil des physiologischen Signals im Wesentlichen unterdrückt ist.

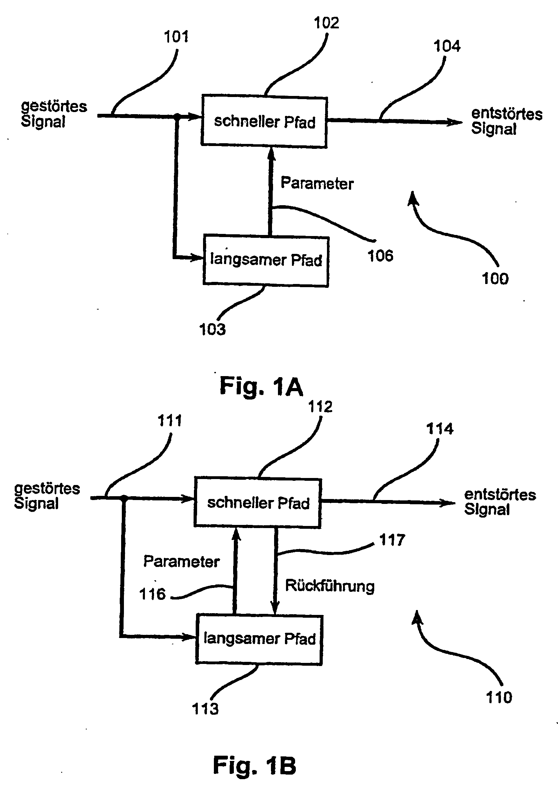

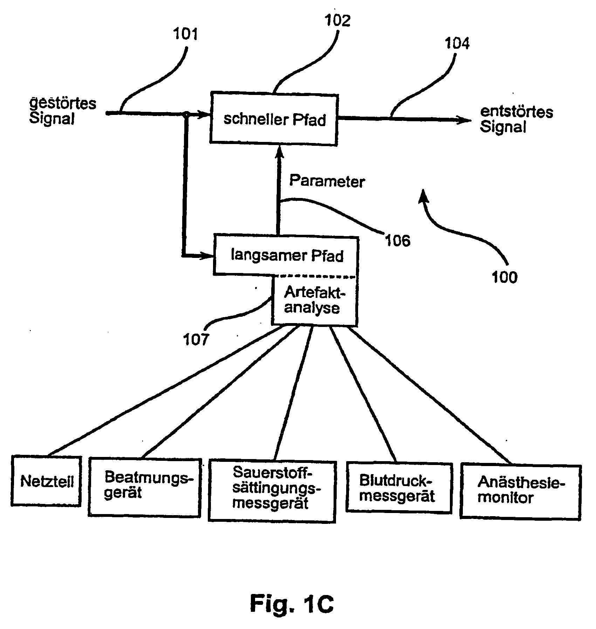

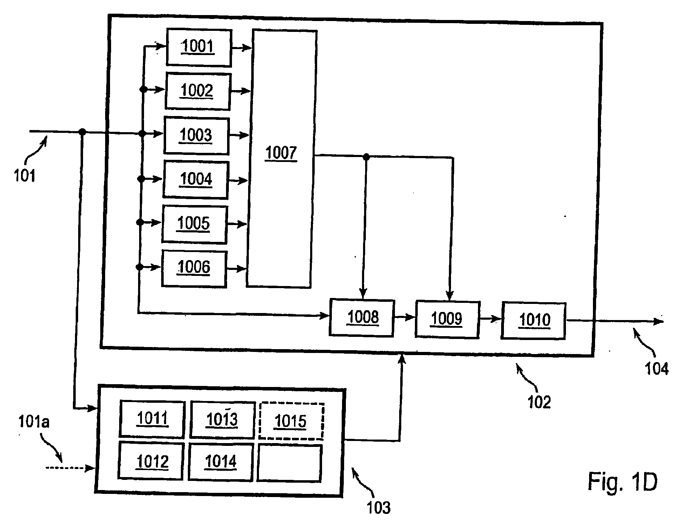

In einem bevorzugten ersten Ausführungsbeispiel (Figuren 1A, 1B, 1C und 1D) der Erfindung wird die Anwendung bei der Ansteuerung eines Beatmungsgeräts auf Basis eines entsprechend gefilterten elektromyographischen Signals (EMG) von einem Patienten dargestellt. In dieser Anwendung ist eine minimale Verzögerungszeit bei der Filterung des elektromyographischen Signals gefordert, so dass die Beatmungssteuerung ohne wesentliche zeitliche Verzögerung auf sich ändernde Beatmungsanforderungen reagieren kann. Bei diesem Ausführungsbeispiel hat die erfindungsgemäße Filterung das Ziel, die durch den Herzschlag des Patienten verursachten EKG-Artefakte - insbesondere die R-Zacken - ohne größere zeitliche Verzögerung zu entfernen, ohne das Nutzsignal zu beeinträchtigen.

Eine herkömmliche lineare Filterung (z.B. Hochpassfilter) hätte entweder eine zu hohe Verzögerungszeit zur Folge oder ein erheblicher Teil der Signalenergie ginge verloren, da die Grenzfrequenz des Hochpassfilters entsprechend hoch eingestellt werden müsste, um die EKG-Artefakte hinreichend zu entfernen.

Das obige Problem wird gelöst, indem bei der Filterung eine Auftrennung des Signalpfades in mehrere separate Signalpfade erfolgt, und zwar in einen schnellen Signalpfad und mindestens einen langsamen Signalpfad. Die Signalpfade greifen auf dasselbe gestörte Eingangssignal zu, das sowohl einen Nutz- als auch einen Störanteil (z.B. EKG-Artefakte) enthält. Das Ergebnis der Filterung im langsamen Signalpfad steht erst nach einer größeren Verzögerungszeit zur Verfügung und wird dann bei der Filterung im schnellen Signalpfad zum Beispiel in Form von Parametern verwendet. Diese Kopplung des langsamen Signalpfades mit dem schnellen Signalpfad ist in bevorzugter Weise geeignet ausgebildet, das funktionale Verhalten des langsamen Signalpfades zu beeinflussen.

Im Sinne der vorliegenden Erfindung ist unter einem schnellen Signalpfad zu verstehen, dass ein Signal den schnellen Pfad vom Eingang bis zum Ausgang um einen Faktor 5 bis 20 mal schneller als den langsamen Signalpfad durchläuft. Zur Differenzierung der Begriffe "schneller Signalpfad" und "langsamer Signalpfad" soll verstanden werden, dass die Signaldurchlaufzeit im schnellen Pfad mindestens um den Faktor 2 kleiner ist als im langsamen Pfad. Dieser Faktor hat in bevorzugten Ausgestaltungen der Erfindung allgemein Werte zwischen 2 und 25, und vorzugsweise zwischen 5 und 20. Beispielhaft und typisch im Sinne der vorliegenden Erfindung für einen schnellen Signalpfad ist eine Durchlaufzeit von 20 Millisekunden, für einen langsamen Signalpfad eine Durchlaufzeit von 200 Millisekunden. Grundsätzlich soll der schnelle Signalpfad schnell auf Änderungen des Eingangssignals reagieren und gleichzeitig an seinem Ausgang eine möglichst glatte Hüllkurve zur Verfügung stellen, die dann zur Ansteuerung eines Gerätes dient. Für die Glättung der Hüllkurve ist natürlich eine gewisse Zeit (Reaktionszeit) erforderlich, die abhängig vom gewünschten Ausmaß der Glättung und der verwendeten Filter variieren kann. Die üblichen Durchlauf- bzw. Reaktionszeiten für den schnellen Pfad liegen daher in einem Bereich von vorzugsweise zwischen etwa 20 ms und etwa 200 ms. Im Gegensatz dazu liegen die Durchlauf- bzw. Reaktionszeiten im langsamen Pfad in einem Bereich von vorzugsweise zwischen 200 ms und etwa 10 s.

Für die Entfernung von EKG- Artefakten aus einem EMG- Nutzsignal sind die Signalpfade an die Signalbestandteile des EKG- Signals und des EMG- Signals angepasst.

Für das EKG- Signal ist eine Abtastung im Bereich von 250 Werten je Sekunde bis 1500 Werten je Sekunde erforderlich und zweckmäßig, für das EMG- Signal ist eine Abtastung im Bereich von 250 Werten je Sekunde bis 1500 Werten je Sekunde erforderlich und zweckmäßig.

Das gefilterte EMG- Nutzsignal soll nach einer Durchlaufzeit für die Filterung im Bereich innerhalb eines Atemzyklus eines Patienten, d.h. in einem Bereich von 0,05 Sekunden bis von 0,25 Sekunden zur weiteren Verwendung, beispielsweise zur Steuerung eines Beatmungsgerätes zur Verfügung stehen. Allgemein kann statt des Beatmungsgeräts auch irgendein anderes externes Gerät mit dem Signalsausgang der Filtervorrichtung gekoppelt sein. Hierbei kann das externe Gerät über eine Signalleitung mit dem langsamen Pfad gekoppelt sein. Grundsätzlich kann der Signalausgang bei einer bevorzugten Ausgestaltung mit dem langsamen Signalpfad gekoppelt sein.

Auf dieser Grundlage ist der schnelle Signalpfad mit einer Durchlaufzeit von 0,02 Sekunden bemessen. Der langsame Signalpfad ist auf Grundlage des zeitlichen Verlaufs der EKG- Artefakte mit einer typischen Durchlaufzeit im Bereich von 0,3 Sekunden bis 1 Sekunden bemessen.

Zu einer deutlichen Verbesserung der Unterdrückung der EKG- Artefakte im EMG- Signal in der Praxis ist es aber durchaus realistisch, die Durchlaufzeit für den langsamen Signalpfad in einem Bereich von 1 Sekunden bis 10 Sekunden zu bemessen.

Das resultierende Ausgangssignal enthält idealerweise nur den Nutzanteil. In Wirklichkeit wird der Störanteil lediglich stärker verringert als der Nutzanteil. Bevorzugt wird mindestens im schnellen Signalpfad ein Filter mit nichtlinearen Eigenschaften verwendet. Im langsamen Signalpfad können die bekannten Methoden der linearen Filterung inklusive adaptiver Filter verwendet werden, da die größere Verzögerungszeit keine Rolle spielt.

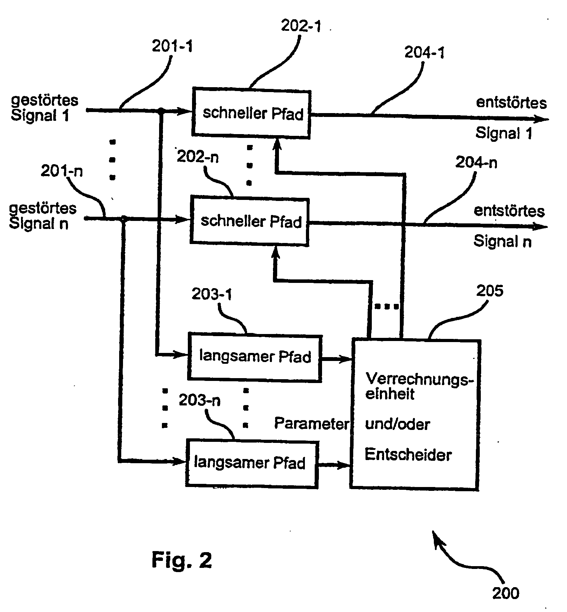

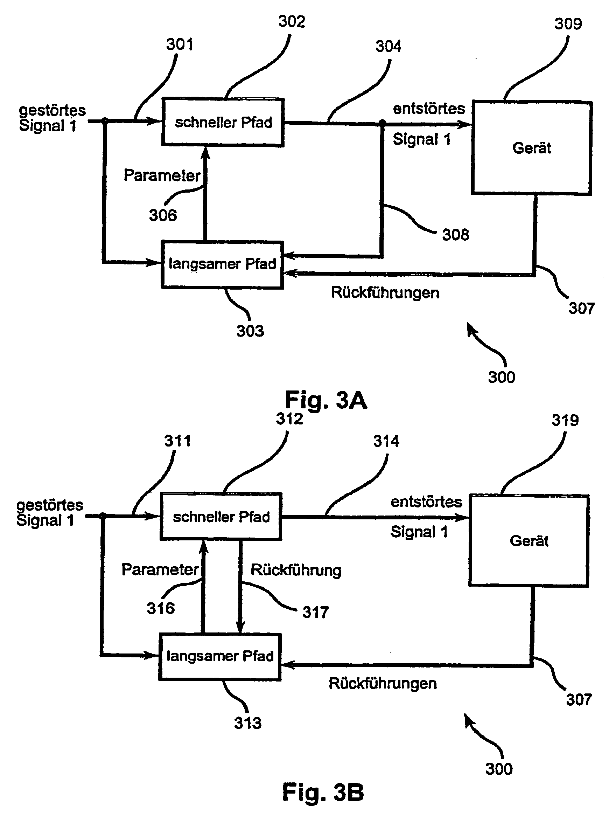

In einem weiteren zweiten Ausführungsbeispiel (Figur 2) erfolgt eine Quervernetzung von Informationen zwischen mehreren Signalpfaden. Somit können Ergebnisse der langsamen Filterung eines Signals auf die schnelle Filterung eines anderen Signals angewendet werden. Dieses zweite Ausführungsbeispiel lässt sich durch eine Replikation und Vernetzung der Signalpfade aus dem ersten Ausführungsbeispiel ableiten, wobei eine zusätzliche Verrechnungs- bzw. Entscheidungseinheit (VE) vorgesehen ist. Alternativ können die langsamen Signalpfade komplett in der Entscheidungseinheit enthalten sein. In einem weiteren dritten Ausführungsbeispiel (Figuren 3A und 3B) gibt es im Vergleich zum ersten Ausführungsbeispiel zusätzliche Rückführungen vom Ausgang des schnellen Signalpfades oder von einem Ausgang des gespeisten externen Geräts auf den langsamen Signalpfad. Hierdurch wird eine automatische Adaption der Signalverarbeitung ermöglicht.

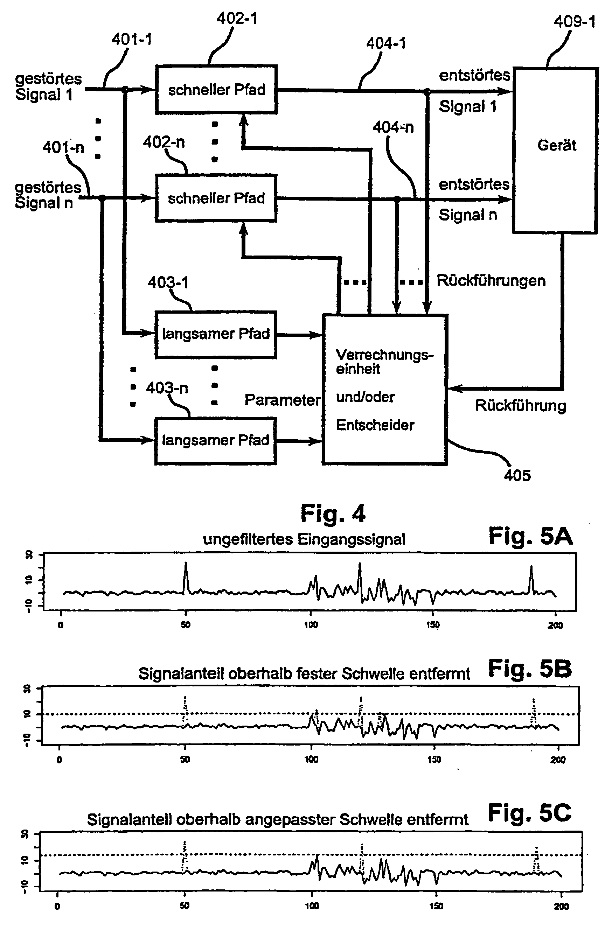

In einem weiteren vierten Ausführungsbeispiel (Figur 4) gibt es im Vergleich zum zweiten Ausführungsbeispiel zusätzliche Rückführungen von den Ausgängen der schnellen Pfade oder von einem Ausgang (bzw. von mehreren Ausgängen) des gespeisten externen Geräts auf die Verrechnungs-/Entscheidungseinheit. Wie in Figuren 3A und 3B wird hierdurch eine automatische Adaption der Signalverarbeitung ermöglicht.

Die vorliegende Erfindung wird nun anhand einiger Ausführungsbeispiele unter Bezugnahme auf die Figuren beschrieben, die verschiedene Ausgestaltungen der erfindungsgemäßen Vorrichtung zur Datenverarbeitung physiologischer Signale bzw. der erfindungsgemäßen Filtervorrichtung zeigen. Obwohl sich die folgende Beschreibung auf eine Vorrichtung zur Datenverarbeitung bzw. auf eine Filtervorrichtung zur Verwendung bei der Steuerung eines Beatmungsgeräts bezieht, kann die erfindungsgemäße Vorrichtung, wie für den Fachmann offensichtlich, auch für die Verarbeitung anderer physiologischer Signale verwendet werden kann, bei denen es darauf ankommt, Störsignale ohne größere zeitliche Verzögerung zu entfernen, ohne das Nutzsignal zu beeinträchtigen.
- Figur 1A: zeigt eine schematische Darstellung von einem ersten Ausführungsbeispiel der vorliegenden Erfindung, das mit dem Ziel der Echtzeitfilterung bei der Ansteuerung eines Beatmungsgerätes verwendet werden kann;
- Figur 1B: zeigt eine Abwandlung der Ausführungsbeispiels aus Figur 1 A;
- Figur 1C: zeigt die Ausgestaltung aus Figur 1A, in der das "langsame Filter" mit einem zusätzlichen Block "Artefakt-Analyse" versehen ist, der Eingänge für Zusatzsignale aufweist;
- Figur 1 D: zeigt Details des "schnellen Pfades" und des "langsamen Pfades" aus Figuren 1A bis 1C;
- Figur 2: zeigt eine schematische Darstellung von einem zweiten Ausführungsbeispiel der vorliegenden Erfindung, bei dem eine Quervernetzung von Informationen zwischen mehreren Signalpfaden erfolgt;
- Figur 3A: zeigt eine schematische Darstellung von einem dritten Ausführungsbeispiel der vorliegenden Erfindung, bei dem im Vergleich zum ersten Ausführungsbeispiel zusätzliche Rückführungen vom Ausgang des schnellen Signalpfades oder vom Ausgangssignal eines externen Geräts auf den langsamen Signalpfad vorgesehen sind;
- Figur 3B: zeigt eine Abwandlung der Ausführungsbeispiels aus Figur 3A;
- Figur 4: zeigt eine schematische Darstellung von einem vierten Ausführungsbeispiel der vorliegenden Erfindung, bei dem im Vergleich zum zweiten Ausführungsbeispiel zusätzliche Rückführungen von den Ausgängen der schnellen Pfade oder vom Ausgangssignal des gespeisten externen Geräts auf die Verrechnungs-/Entscheidungseinheit vorgesehen sind;
- Figuren 5A bis 5C: zeigen beispielhaft Darstellungen eines durch das EKG gestörten elektromyographischen Signals zur Erläuterung der Funktion des ersten Ausführungsbeispiels aus Figuren 1A bis 1C; und
- Figur 6: ist eine Darstellung, in der eine detaillierte Ausgestaltung des schnellen und des langsamen Signalpfades des Ausführungsbeispiels aus Figur 1 B gezeigt ist.

Figur 1A zeigt eine Darstellung von einem ersten Ausführungsbeispiel einer Filtervorrichtung 100 der vorliegenden Erfindung. Die Filtervorrichtung 100 weist einen schnellen Signalpfad 102 und mindestens einen langsamen Signalpfad 103 auf, die parallel angeordnet sind. Die Signalpfade 102 und 103 greifen auf dasselbe gestörte Eingangssignal 101 zu, das sowohl einen Nutz- als auch einen Störanteil enthält. Das Ergebnis der Filterung im langsamen Signalpfad 103 steht erst nach einer größeren Verzögerungszeit zur Verfügung und wird dann bei der Filterung im schnellen Signalpfad 102 zum Beispiel in Form von Parametern verwendet. Hierzu ist der langsame Signalpfad 103 über eine Signalleitung 106 mit dem schnellen Signalpfad 102 gekoppelt. Das resultierende Ausgangssignal 104 enthält idealerweise nur den Nutzanteil. In Wirklichkeit wird der Störanteil aber lediglich stärker verringert als der Nutzanteil. Bevorzugt wird mindestens im schnellen Signalpfad 102 ein Filter mit nichtlinearen Eigenschaften verwendet. Im langsamen Signalpfad 103 können die bekannten Methoden der linearen Filterung inklusive adaptiver Filter verwendet werden, da die größere Verzögerungszeit keine Rolle spielt.

Die Filtervorrichtung 100 aus Figur 1A findet vorzugsweise bei der Echtzeitfilterung physiologischer Signale Anwendung, beispielsweise bei der Filterung elektromyographischer Signale von einem Patienten, die zur Ansteuerung eines Beatmungsgeräts auf Basis der entsprechend gefilterten elektromyographischen Signale verwendet werden. Wie vorstehend erläutert, erfordert eine solche Beatmungssteuerung eine minimale Verzögerungszeit bei der Filterung des elektromyographischen Signals, was durch die Filtervorrichtung aus Figur 1A erreicht wird.

Zum Beispiel kann die Filtervorrichtung 100 aus Figur 1A mit dem Ziel verwendet werden, die durch den Herzschlag des Patienten verursachten EKG-Artefakte (z.B. die R-Zacken) ohne größere zeitliche Verzögerung zu entfernen, ohne dabei das Nutzsignal zu beeinträchtigen.

Figur 1B zeigt eine Abwandlung der in Figur 1A gezeigten Filtervorrichtung. Die Filtervorrichtung 110 hat ebenfalls einen schnellen Signalpfad 112 und mindestens einen langsamen Signalpfad 113, die parallel angeordnet sind und auf dasselbe gestörte Eingangssignal 111 zugreifen. Das Ergebnis der Filterung im langsamen Signalpfad 113 steht erst nach einer größeren Verzögerungszeit zur Verfügung und wird dann bei der Filterung im schnellen Signalpfad 112 zum Beispiel in Form von Parametern verwendet. Hierzu ist der langsame Signalpfad 113 über eine Signalleitung 116 mit dem schnellen Signalpfad 112 gekoppelt. Ferner wird ein Signal vom schnellen Signalpfad 112, das nicht das Ausgangssignal 114 sein muss, über eine Signalleitung 117 an den langsamen Signalpfad 113 zurückgeführt, um auf diese Weise das funktionale Verhalten des langsamen Signalpfades 113 zu beeinflussen. Mit Hilfe dieser Signalrückführung bzw. Signalrückkopplung lässt sich beispielsweise die Geschwindigkeit des langsamen Signalpfades 113 innerhalb bestimmter Grenzen an die jeweilige Anwendung anpassen.

Figur 1C zeigt eine Ausgestaltung aus Figur 1A, in der der langsame Signalpfad 103 mit einem zusätzlichen Block 107 "Artefakt-Analyse" versehen ist, der Eingänge für Zusatzsignale aufweist. Diese Zusatzsignale stammen beispielsweise von externen Quellen (Quellen für Störsignale) bzw. externen Geräten, die Störsignale in Form von Spannungen und Strömen unterschiedlicher Frequenzen erzeugen. Beispiele solcher Geräte sind das Beatmungsgerät selbst, Netzteile des Beatmungsgeräts oder anderer Geräte, Anästhesiegeräte, Gasmonitore, Blutdruckmessgeräte, Sauerstoffsättigungsmessgeräte (S_{P}O₂). Der Block 107 kann aber auch andere Sensorsignale erhalten, wie zum Beispiel Signale von Dehnungssensoren (z.B. für Abdomen/Thorax), Beschleunigungssensoren, Sensoren zur Messung der Thoraximpedanz, etc. In Figur 1C sind diese beispielhaften Geräte als Störsignalquellen eingezeichnet. Von diesen Geräten stammende Zusatzsignale können aber auch - jedes für sich oder in Kombination oder in Kombination mit dem EKG-Signal - verwendet werden, um bestimmte patientenbedingte Situationen (z.B. Sauerstoffmangel, Blutdruckabfall, Husten, Schluckauf, Seufzer, Atemaussetzer und/oder Atemfrequenzschwankungen) zu erkennen und die Steuerung beispielsweise des Beatmungsgeräts entsprechend anzupassen oder zu modifizieren. Beispiele von verwendeten Zusatzsignalen sind Flow (Atemstrom), Druck (Atemdruck), PEEP (Positive EndExpiratory Pressure), RR-, CO₂-, O₂- und S_{P}O₂-Konzentration, NIV (Non-Invasive Ventilation), BP (Blood Pressure), Anästhesiegas-Konzentration, Netzspannung und Netzfrequenz. Aber auch die Netzfrequenz oder die Beatmungsfrequenz selbst sind beispielsweise zur Adaption und Auslegung der Filterfrequenzen oder bei der Anwendung von Korrelation-Wavelet-Filterung vorteilhaft im langsamen Pfad zu kennen, um das Nutzsignal aus dem gestörten Signal im schnellen Pfad zu extrahieren. Es ist offensichtlich, dass der Artefakt-Analyse-Block 107 auch in den anderen Ausführungsbeispielen der Figuren 1B, 2, 3A, 3B und 4 verwendet werden kann.

Figur 1D zeigt Details des schnellen Signalpfades 102 und des langsamen Signalpfades 103 aus Figuren 1A und 1C, wobei die dargestellten Komponenten aber auch in den jeweiligen Pfaden aus Figur 1B enthalten sein können. Wie in Figur 1D zu sehen, liegt an den Eingängen sowohl des schnellen Pfades 102 wie auch des langsamen Pfades 103 ein gestörtes EMG- oder sEMG-Signal 101 an (EMG = Elektromyogramm / sEMG = Surface EMG). Im schnellen Pfad 102 wird dieses gestörte EMG-Signal beispielsweise einem QRS-Detektor 1001, einem P-Wellen-Detektor 1002, einem Netzartefakt-Detektor 1003, einem Crosstalk-Detektor 1004, einem Schluckauf-Detektor 1005 oder einem oder mehreren anderen Detektoren 1006 zugeführt, die in dieser Beschreibung offenbart sind, und gleichzeitig einem Baseline-Filter 1008 zugeführt. Die Ausgaben dieser Detektoren werden einem Logikfunktion-Block und/oder Fuzzy-Controller 1007 zugeführt, dessen Ausgabe dem Baseline-Filter 1008 und einem Artefakt-Entfernung-Block 1009 zugeführt wird, wobei dem Artefakt-Entfernung-Block außerdem die Ausgabe des Baseline-Filters zugeführt wird. Die Ausgabe des Artefakt-Entfernung-Blocks wird anschließend der Hüllkurven-Berechnung 1010 zugeführt, die das entstörte EMG- bzw. sEMG 104 ausgibt.

In Figur 1D ist ferner gezeigt, dass das gestörte EMG- bzw. sEMG-Signal 101 auch dem langsamen Signalpfad 103 zugeführt wird, der die vorstehend erläuterten Komponenten enthält und dessen Ausgabe mit dem schnellen Signalpfad 102 gekoppelt ist. Ferner ist dargestellt, dass dem langsamen Signalpfad optional verschiedene pneumatische Signal 101a zugeführt werden können.

Wie in Figur 1D dargestellt, enthält der Block "schneller Pfad" 102 für verschiedene mögliche Artefakte einen jeweiligen Detektor und einen Logikblock, in dem das zeitliche Auftreten der Artefakte logisch verknüpft wird. Die Verknüpfung kann beispielsweise durch logische Kombination von binären Signalen erfolgen, die das detektierte oder/und erwartete Auftreten von spezifischen Artefakten beschreiben. Alternativ können graduelle zeitliche Verläufe verwendet werden, die die Wahrscheinlichkeit des Auftretens von spezifischen Artefakten beschreiben. Zur Kombination solcher Verläufe bietet sich beispielsweise die gezeigte Fuzzy-Logik an.

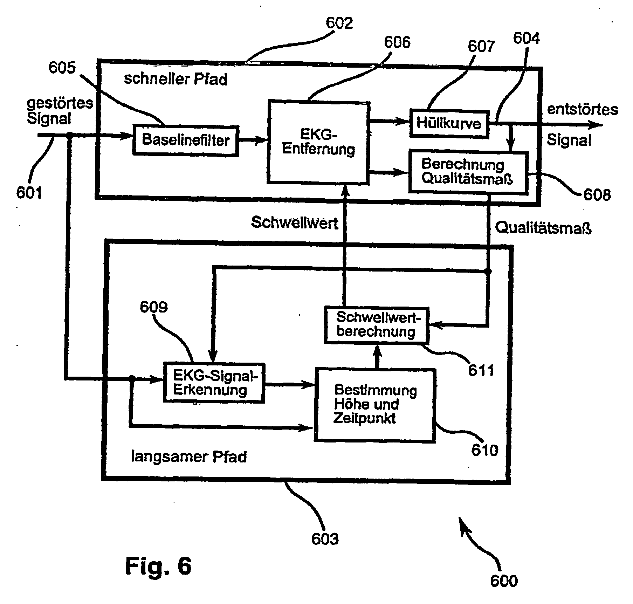

Wie vorstehend bereits erwähnt, enthält der Block "schneller Pfad" (ähnlich wie in Figur 6) weiterhin einen Baseline-Filter, einen Block zur Artefaktentfernung sowie einen Block, der die Hüllkurvenberechnung durchführt. Die Blöcke "Baseline-Filter" und Artefaktentfernung" werden von dem Ausgangssignal des Logikblocks moduliert. Beispielsweise werden Parameter des Baseline-Filters (z.B. des Rangordnungsfilters) abhängig von dem Grad der Artefakterwartung angepasst. Ähnlich wird der Block "Artefaktentfernung" nur aktiv, wenn er entsprechend von dem Logikblock angesteuert wird.

Alle Blöcke des schnellen Pfades erhalten Zugriff auf die Parameter, die der langsame Pfad 103 ermittelt hat, wie bereits erläutert wurde. So können die Detektoren optional an die jeweiligen Artefakteigenschaften angepasst werden. Diese Parameter sind zum Beispiel EKG-Parameter 1011, Crosstalk-Parameter 1012, Schluckaufparameter 1013, Netzartefaktparameter 1014, Parameter für Spontanatmung und weitere Parameter.

Nach der Artefaktentfernung können, wie nachfolgend unter Bezugnahme auf Figur 6 beschrieben wird, Qualitätsmaße berechnet werden, welche zur Verbesserung der Parameterbestimmung im langsamen Pfad verwendet werden können. Wenn sich beispielsweise nach der erfolgten Artefaktentfernung herausstellt, dass die Artefakte nicht vollständig entfernt wurden, könnten die entsprechenden Artefaktparameter angepasst werden, um im Folgeschritt eine verbesserte Entfernung zu erreichen. Die Berechnung der Qualitätsmaße muss nicht im schnellen Pfad erfolgen. Wird sie im langsamen Pfad durchgeführt, werden dafür jedoch Signale aus dem schnellen Pfad benötigt. Dies entspricht der Rückführung 317 in Figur 3B, was nachfolgend im größeren Detail beschrieben wird.

Figur 2 zeigt ein zweites Ausführungsbeispiels der Filtervorrichtung der vorliegenden Erfindung. Bei dieser Filtervorrichtung 200 erfolgt allgemein eine Quervemetzung von Informationen zwischen mehreren Signalpfaden. Die Filtervorrichtung hat eine Mehrzahl von Signaleingängen 1 bis n, die mit Bezugszeichen 201-1 bis 201-n bezeichnet sind, um mehrere verschiedene physiologische Eingangssignale (z.B. elektromyographische Signale, EKG-Signal, Atemfrequenz, etc.) von einem Patienten zu empfangen, die jeweils sowohl einen Nutz- als auch einen Störanteil enthalten. Jedes dieser Eingangssignale wird sowohl einem langsamen Signalpfad 203-1 bis 203-n als auch einem schnellen Signalpfad 202-1 bis 202-n zugeführt. Die schnellen Signalpfade enthalten jeweils ein vorzugsweise nichtlineares Filter, um eine schnelle Filterung zu bewirken, und die langsamen Signalpfade enthalten jeweils ein vorzugsweise lineares Filter, um eine langsame Filterung zu bewirken. An den Ausgängen der jeweiligen schnellen Signalpfade 202-1 bis 202-n liegen jeweils die im Wesentlichen entstörten Signale 204-1 bis 204-n an. Die Ausgänge der langsamen Signalpfade 203-1 bis 203-n sind jeweils mit einer Verrechnungs-/Entscheidungseinheit 205 verbunden, deren Ausgänge jeweils mit schnellen Signalpfaden 202-1 bis 202-n gekoppelt sind. Auf diese Weise lassen sich die Ausgänge der langsamen Signalpfade auf beliebige Weise mit gewünschten schnellen Signalpfaden gekoppelt werden. Somit können Ergebnisse der langsamen Filterung eines Signals beliebig auf die schnelle Filterung eines anderen Signals angewendet werden.

In Figuren 3A und 3B ist ein drittes Ausführungsbeispiel der vorliegenden Erfindung gezeigt. Die Filtervorrichtungen 300 und 310 aus Figuren 3A und 3B sind in ihrem grundsätzlichen Aufbau den Filtervorrichtungen 100 und 110 aus Figuren 1A und 1B ähnlich. Beide Filtervorrichtungen empfangen an ihren Eingängen 301 bzw. 311 ein gestörtes (physiologisches) Signal, das einem schnellen Signalpfad 302/312 und einem dazu parallel geschalteten langsamen Signalpfad 303/313 zugeführt. Der Ausgang 304/314 des schnellen Signalpfades liegt ein nahezu entstörtes Signal an, das einem Gerät 309/319 (z.B. einem Beatmungsgerät) zugeführt wird. Wie in Figur 3A zu sehen, wird das Ausgangssignal 304 als Rückkopplungssignal an den langsamen Signalpfad 303 zurückgeführt. In Figur 3B ist eine alternative Ausgestaltung gezeigt, in der ein Signal von einer beliebigen Stelle des nichtlinearen Filters des schnellen Signalpfades 312 an eine beliebige Stelle des linearen Filters des langsamen Signalpfades 313 zurückgeführt wird. Im Vergleich zum ersten Ausführungsbeispiel ist in den Ausgestaltungen der Figuren 3A/3B eine zusätzliche Rückführung von einem Ausgang des gespeisten externen Gerätes 309/319 auf den langsamen Signalpfad 303/313 vorgesehen. Hierdurch wird eine automatische Adaption der Signalverarbeitung ermöglicht.

Das in Figur 4 gezeigte Ausführungsbeispiel ist ähnlich dem in Figur 2 gezeigten Ausführungsbeispiel mit der Ausnahme, dass ein externes Gerät 409-1 mit den Ausgängen 404-1 bis 404-n der schnellen Signalpfade 402-1 bis 402-n gekoppelt ist. Ein Ausgang dieses Geräts 409-1 ist mit einem Eingang der Verrechnungs-/Entscheidungseinheit 405 gekoppelt. Ferner sind die Ausgänge der schnellen Signalpfade 402-1 bis 402-n mit der Verrechnungs-/ Entscheidungseinheit 405 rückgekoppelt.

Figuren 5A bis 5C zeigen beispielhaft Darstellungen eines durch das EKG gestörten elektromyographischen Signals zur Erläuterung der Funktion des ersten Ausführungsbeispiels. Im schnellen Signalpfad ist ein nichtlineares Filter basierend auf einer Ausreißeranalyse (Vergleich mit fester Schwelle, gestrichelte horizontale Linie) realisiert, um die R-Zacken des EKGs zu entfernen (Fig. 5A, t=50, t=120, t=290). Das Ergebnis der Filterung zeigt Fig. 5B: die gepunkteten Signalanteile wurden entfernt. Neben den R-Zacken ist jedoch auch ein Teil des Nutzsignals betroffen, das ebenfalls entfernt wird (t=105, t=130).

Um besser zwischen Nutzsignal und R-Zacken unterscheiden zu können, wird für Fig. 5C nun anstelle einer festen Schwelle der langsame Pfad mit einem Standard-QRS-Erkennungsverfahren (z.B. Pan-Tompkins [siehe J. Pan and WJ. Tompkins: A real time QRS detection algorithm, IEEE Transactions on Biomedical Engineering, Vol. BME-32, No. 3, March 1985]) versehen, der als Parameter für den schnellen Pfad die Höhe der letzten R-Zacke liefert. Diese wird im schnellen Pfad für die Unterscheidung zwischen Nutzsignal und R-Zacke verwendet, indem die Schwelle (gestrichelte Linie) nachgestellt wird.

Figur 6 ist eine detaillierte Darstellung eines bevorzugten Ausführungsbeispiels einer Filtervorrichtung 600 mit einem langsamen und einem schnellen Signalpfad. Der schnelle Signalpfad 602 enthält ein Baselinefilter 605, einen Block 606 zum Entfernen eines EKG-Signals (bzw. Ausreißer), eine Hüllkurvenberechnungseinheit 607 und eine Einheit 608 zum Berechnen eines Qualitätsmaßes. Das gestörte Eingangssignal 601 wird einem Eingang des Baselinefilters 605 zugeführt, dessen Ausgang mit einem ersten Eingang des Blocks 606 zum Entfernen des EKG-Signals gekoppelt ist. Dabei durchläuft das Eingangssignal das Baselinefilter, das tieffrequente Anteile (z.B. die R-Zacke des im Eingangssignal enthaltenen EKG-Signals) des Eingangssignals entfernt und als linearer Filter mit Hochpasscharakteristik oder als nichtlinearer Filter ausgelegt sein kann. Der Block 606 zur EKG-Entfernung hat zwei Ausgänge, von denen ein erster Ausgang mit einem Eingang der Hüllkurvenberechnungseinheit 607 und ein zweiter Ausgang mit einem ersten Eingang der Einheit 608 zum Berechnen eines Qualitätsmaßes gekoppelt sind. Der Ausgang der Hüllkurvenberechnungseinheit 607 liefert das im Wesentlichen entstörte Ausgangssignal 604, das sowohl am Ausgang des schnellen Signalpfades 602 (und somit am Ausgang der Filtervorrichtung) zur Verfügung steht als auch einem zweiten Eingang der Einheit 608 zugeführt wird. Der Block 606 zum Entfernen eines EKG-Signals hat einen zweiten Eingang, dem ein dynamischer Schwellwert zugeführt wird, der von einer Einheit 611 zur Schwellwertberechnung des langsamen Signalpfades 603 geliefert wird, was nachfolgend in größerem Detail erläutert wird. Ferner hat die Einheit 608 zum Berechnen eines Qualitätsmaßes einen Ausgang, auf dem ein Signal an einen ersten Eingang der Einheit 611 zur Schwellwertberechnung und an einen zweiten Eingang einer EKG-Signalerkennungseinheit 609 des langsamen Signalpfades 603 ausgegeben wird, wobei dieses Signal dem Qualitätsmaß entspricht, auf Basis dessen die Schwellwertberechnung und die EKG-Signalerkennung durchgeführt wird.

Das Baselinefilter 605 des schnellen Signalpfades 602 hat allgemein die Aufgabe, eine Grundlinie ("Baseline") des gestörten Eingangssignals 601 zu beseitigen. Die Grundlinie besteht typischerweise aus einem Gleichanteil (Frequenz gleich Null) und niederfrequenten Anteilen. Dieses Baselinefilter 605 ist hier als nichtlineares Filter ausgelegt, um einerseits möglichst geringe Signalverzögerungszeiten zu erreichen und um andererseits die Form des EKG-Signals (bzw. Ausreißer) so wenig wie möglich zu verändern. Dies ist insbesondere deshalb ein Problem, weil die R-Zacke des EKGs üblicherweise eine Dreieckform aufweist, die einen Gleichanteil enthält. Dieser Gleichanteil wird durch die bekannten linearen Filter (hier Hochpassfilter) grundsätzlich hin zu einem bipolaren Signal verändert, da die R-Zacke durch die lineare Filterung einer Faltung mit der Impulsantwort des Hochpassfilters unterworfen wird. Das in dem bevorzugten Ausführungsbeispiel verwendete nichtlineare Filter basiert auf der Benutzung eines Rangordnungsfilters. Signalfilterungen auf Basis von Rangordnungsfiltern sind, anders als gleitende oder auf Basis vordefinierter Anzahlen von Messwerten durchgeführte Filterungen in der Art einer arithmetischen Mittelwertbildung, unempfindlich für Ausreißer im Signal. Rangordnungsfilter im Sinne der vorliegenden Erfindung sind beispielsweise Medianfilter, Extremwertfilter oder sogenannte "Smoothing"- Filter oder "einen Messwert auswählende Filter", die aus einer vorbestimmten, in das Filter eingespeisten Messwertanzahl nach einer Rangordnung einen bestimmten Wert das Filter passieren lassen, also beispielsweise den größten Messwert, den zweit- größten Messwert, den kleinsten Messwert, den zweit- kleinsten Messwert, einen mittleren Wert aus der Messwertanzahl oder einen nächstliegend größeren oder nächstliegend kleineren Wert zum mittleren Wert aus der Messwertanzahl. Solche Filter sind auch als folgende Filtertypen "1 = aus- 3 - Filter", "1 - aus- 5 - Filter", "1 - aus- n - Filter", "minimaler Wert-aus- 5 - Filter", "maximaler Wert - aus- 5 - Filter", "Minimum - aus- n - Filter", "Maximum - aus- n - Filter", "mittlerer Wert - aus- n - Filter" bekannt (oft auch "Medianfilter" genannt) und werden beispielsweise vielfach im Bereich der Bildbearbeitung eingesetzt. Die beschriebenen Filtertypen sind im Rahmen der vorliegenden Erfindung nur in einer beispielhaften Aufzählung genannt, welche in keiner Weise einschränkend auf die Bezeichnung eines Rangordnungsfilters, sondern vielmehr sind Modifikationen und Kombinationen dieser Typen im Sinne der vorliegenden Erfindung mit umfasst. Kennzeichnend für solche Rangordnungsfilter ist, dass ein Messwert das Filter unverändert passieren kann, anders als bei anderen Filtern, in denen mehrere Messwerte zu einem neuen Wert am Filterausgang führen, wie beispielsweise bei einer arithmetischen Mittelwertfilterung. Durch Differenzbildung mit dem ungefilterten Signal entsteht ein Signal, das diese Ausreißer noch enthält, aber dennoch die Grundlinie entfernt. Ausreißer in Form der R-Zacke des EKG-Signals werden dabei in ihrer Form erhalten, aber mit verminderter Höhe und Länge. Diese Verminderung der Höhe und Länge hängt mit der Verzögerungszeit des Baselinefilters 605 zusammen.

Der Block 606 zur EKG-Entfernung soll die Signalanteile entfernen, die dem EKG zuzuordnen sind. Dadurch, dass durch die Baselinefilterung des Baselinefilters 605 insbesondere die R-Zacke in ihrer Form nicht verzerrt wird, kann mittels einer Schwellwertdetektion auf einfache Weise die R-Zacke des EKGs erkannt werden. Ein geeigneter Schwellwert, der durch die Einheit 611 zur Schwellwertberechnung des langsamen Signalpfades 603 geliefert wird, ist allerdings für eine effektive Erkennung bei gleichzeitig geringer Fehlerquote erforderlich. Ist eine R-Zacke (bzw. ein Ausreißer) erkannt, kann das Signal an dieser Stelle z.B. auf Null oder auf einen anderen geeigneten Wert gesetzt werden. Dies erfolgt erfindungsgemäß in einer solchen Weise, dass die nachfolgende Hüllkurvenberechnung nicht wesentlich beeinträchtigt wird. Zum einen sollte der Zeitabschnitt des Ausreißers bzw. der Zeitabschnitt, bei dem das Signal auf einen geeigneten Wert gesetzt wird, so kurz wie möglich sein, damit keine Nutzsignalanteile ersetzt werden. Bei der vorliegenden Filtervorrichtung haben die genannten nichtlinearen Filter gegenüber linearen Filtern den Vorteil, dass es kein Nachschwingen ("Klingeln") gibt, was dazu führt, dass Ausreißer durch das Baselinefilter 605 nicht zeitlich verlängert werden. Zum anderen muss die Berechnung der Hüllkurve während der Zeit des entfernten Ausreißers so erfolgen, dass sich das Hüllkurvensignal stetig - und nicht sprunghaft - verändert. Bevorzugt wird die Hüllkurve während der Zeit des entfernten Ausreißers extrapoliert, um einen möglichst glatten Hüllkurvenverlauf zu erzielen.

Für eine Anwendung zur Steuerung eines Beatmungsgeräts ist die Berechnung der Hüllkurve sinnvoll, um daraus einen Triggerzeitpunkt zu generieren. Die Information, z.B. an welchen Stellen Ausreißer erkannt wurden, können in der Hüllkurvenberechnungseinheit 607 benutzt werden, etwa um einen glatten Hüllkurvenverlauf zu garantieren.

Aus den Signalen der EKG-Entfernung und/oder der Hüllkurvenberechnung sowie aus internen Variablen der EKG-Entfernung lässt sich ein Qualitätsmaß errechnen, das die Qualität dieser Stufe charakterisiert. Dieser Qualitätsmaß-Parameter kann in dem langsamen Signalpfad 603 benutzt werden. Das Qualitätsmaß kann z.B. angeben, mit welcher Wahrscheinlichkeit der Ausreißer zu Recht erkannt wurde - also wie sicher die Entscheidung zur Eliminierung des Ausreißers war. Alternativ kann das Qualitätsmaß ein Signal- zu Rausch-Verhältnis darstellen.

Wie in Figur 6 gezeigt, enthält der langsame Signalpfad 603 eine EKG-Signalerkennungseinheit 609 (bzw. - allgemeiner ausgedrückt - eine Einheit zur Ausreißererkennung), eine Einheit 610 zur Bestimmung der Höhe und des Zeitpunkts des EKGs bzw. des Ausreißers sowie eine Einheit 611 zur Schwellwertberechnung. Die EKG-Signalerkennungseinheit 609 hat einen ersten Eingang, an dem das gestörte Eingangssignal 601 anliegt, und einen zweiten Eingang, dem das Parametersignal des Qualitätsmaßes von der Einheit 608 zum Berechnen des Qualitätsmaßes zugeführt wird. Die EKG-Signalerkennungseinheit 609 hat ferner einen Ausgang, der mit einem ersten Eingang der Einheit 610 gekoppelt ist. Die Einheit 610 hat einen zweiten Eingang, an dem ebenfalls das gestörte Eingangssignal 601 anliegt, und einen Ausgang, der mit einem zweiten Eingang der Einheit 611 gekoppelt ist.

Die EKG-Signalerkennungseinheit 609 setzt typischerweise ein Standardverfahren ein, wie z.B. einen Pan-Tompkins-Algorithmus. Dabei spielt die damit verbundene Signalverzögerungszeit eine untergeordnete Rolle. Insbesondere lassen sich zur Filterung relativ langsame lineare Filter verwenden. Die Einheit 609 liefert üblicherweise den Zeitpunkt z.B. für das Maximum des EKGs, das normalerweise dem R-Peak zuzuordnen ist. Dieser Zeitpunkt kann jedoch aufgrund der internen Signalverarbeitung in der EKG-Signalerkennung nicht exakt genug bestimmt werden. Um den genauen Zeitpunkt des Maximums und den entsprechenden Wert zu erhalten, erfolgt in der Einheit 610 zur Bestimmung der Höhe und des Zeitpunkts des EKGs ein Vergleich mit dem Originalsignal. Diese Information dient dann bei der Schwellwertberechnung zur Bestimmung eines geeigneten Schwellwertes für die EKG-Entfernung im schnellen Signalpfad 602.

Das Qualitätsmaß, das im schnellen Signalpfad 602 berechnet wurde, lässt sich dann noch nutzen, um die EKG-Signalerkennung und die Schwellwertberechnung zu verbessern.

Vorstehend wurden Ausgestaltungen beschrieben, die auf das Entfernen von EKG-Artefakten gerichtet ist. Zusätzlich oder alternativ kann die Filtervorrichtung der vorliegenden Erfindung ausgestaltet sein, um Netzstörungen von 50 bzw. 60 Hz in einem Wechselspannungsnetz mit 230V bzw. 110V aus dem Eingangssignal herauszufiltern. Auch hier erfolgt die Signalverarbeitung in mehr als einem Pfad, d.h. in mindestens einem schnellen Signalpfad (entsprechend der Anforderung an möglichst kurze Verzögerung) und mindestens einem langsamen Pfad, wobei die Ergebnisse der Berechnungen des langsamen Pfades erst später (z.B. nach Beendigung des aktuellen Atemzuges) verfügbar sein müssen.

Zusammenfassend lässt sich feststellen, dass in dem mindestens einen langsamen Signalpfad bevorzugt folgende Signalparameter ermittelt werden, die im schnellen Signalpfad für die Signalverarbeitung verwendet werden:
- EGK-Parameter zur Quantifizierung des Grades der Beeinflussung des physiologischen Signals durch Herzartefakte z.B. für die Entscheidung, ob QRS-Erkennung und QRS-Entfernung überhaupt durchzuführen sind
- weitere spezifische EKG-Parameter, z.B. Parameter der R-Zacken, der P-Wellen, der QRS-Komplexe, dabei jeweils
   - individuelle und mittlere Amplitudenhöhe, -breite oder -leistung der Artefakte sowie die Streuung (ggf. zusätzlich andere Parameter der Signalamplitudenverteilung)
   - individueller und mittlerer Abstand zwischen den Artefakten sowie die Streuung (ggf. zusätzlich andere Parameter der Signalamplitudenverteilung)
   - individuelle und mittlere Amplitudenhöhe oder -leistung der Signalabschnitte zwischen den Artefakten sowie deren Streuung (ggf. zusätzlich andere Parameter der Signalamplitudenverteilung)
   - Erwartungsfenster (ja/nein) für das erwartete Auftreten von spezifischen Artefakten bzw. des Nutzsignals
- Schluckparameter, z.B. Häufigkeit des Auftretens und der Intensität des Artefakts
- Crosstalk-Parameter zur Quantifizierung des Grades der Störung des physiologischen Signals durch Übersprechen (elektrische Aktivität anderer Muskeln) oder Bewegung. Dies kann durch Analysen der Korrelation des physiologischen Signals mit pneumatischen Signalen (z.B. Atemwegsfluss) erfolgen.
- Netzartefakte-Parameter zur Quantifizierung des Grades der Störung des physiologischen Signals durch Netzartefakte (50/60 Hz), z.B. für die Erkennung von gelockerten oder sogar abgefallenen Elektroden
- Parameter für Spontanatmung, z.B. Atemrate, Atemzugsdauer, Dauer der Inspiration, Dauer der Exspiration, dabei jeweils der individuelle und mittlere Wert sowie die Streuung (ggf. andere Parameter der Verteilung), z.B. für die Einstellung der Parameter der Hüllkurvenberechnung
- alternativ: graduelle zeitliche Verläufe, die die Wahrscheinlichkeit des Auftretens von spezifischen Artefakten bzw. des Nutzsignals beschreiben

Es sei angemerkt, dass die obigen Parameter in Figur 1D im Block "langsamer Pfad" dargestellt sind.

Diese Parameter dienen dazu, in dem mindestens einen schnellen Signalpfad mindestens eine (konstante oder dynamisch veränderliche) Schwelle so einzustellen, dass die Artefakte vom übrigen Nutzsignal leicht zu unterscheiden sind, und daraufhin die Artefakte aus dem Nutzsignal zu entfernen.

Grundsätzlich erfolgt die Entfernung der Artefakte mit einem (ggf. dynamischen) Vorlauf und einem (ggf. dynamischen) Nachlauf. Das heißt, das Zeitfenster, das den Artefakt beinhaltet, ist entweder konstant oder abhängig von den im langsamen Signalpfad ermittelten Parametern der Artefakte (z.B. der Breite oder/und Höhe). Für Artefakte, die z.B. so klein sind, dass sie mittels einer Schwelle nicht präzise detektiert werden können (z.B. P-Wellen), ist es möglich, mittels eines aus dem langsamen Pfad bestimmten Erwartungsfensters (oder graduellen Verlaufs der Wahrscheinlichkeit) den Artefakt hart auszuschneiden und ihn durch ein simuliertes Nutzsignal zu ersetzen oder durch Adaption von Signalverarbeitungsverfahren anderweitig unwirksam zu machen. Letzteres kann durch Umschalten der Filtercharakteristik oder durch graduelle Anpassung der Filterparameter erfolgen - innerhalb des Erwartungsfensters oder abhängig vom Wahrscheinlichkeitsverlauf.

Schließlich wird für das artefaktbereinigte Nutzsignal ein Maß bestimmt, welches den Grad der elektrischen Muskelaktivität repräsentiert. Hierfür können die Hüllkurve, ein Zähler für Signal-Nulldurchgänge, der Verlauf der RMS-Werte oder ein anderes Leistungsmaß verwendet werden. Das Leistungsmaß des artefaktbereinigten Nutzsignals dient zum Monitoring der Atemaktivität und/oder zur Steuerung der Beatmung durch ein Beatmungsgerät.

Die in der Figur 1B beschriebene Signalfilterung ist an dieser Stelle in einer besonderen erfindungsgemäßen Ausführungsform eines erfindungsgemäßen Verfahrens auf Basis programmierten Quellcodes für eine Implementierung in Software nochmals dargestellt.

In einem Verfahren zur Echtzeitfilterung physiologischer Signale mit einem Nutzanteil und einem Störanteil,
- wird an einem Signaleingang eines schnellen Signalpfades 112 und parallel und zeitlich gleichzeitig dazu in einen Signaleingang eines langsamen Signalpfad 113 ein gestörtes physiologisches Signal 111 als Eingangssignal eingespeist,
- wird im schnellen Signalpfad eine 112 eine schnelle Filterung durchlaufen,
- wird im langsamen Signalpfad 113 eine langsame Filterung durchlaufen,
- wird ein Parameter 116 des langsamen Signalpfades 113 in den schnellen Signalpfad 112 eingespeist,
- wird ein Rückführungssignal 117 des schnellen Signalpfad 113 in den langsamen Signalpfad 112 zurück geführt,
- liegt ein entstörtes physiologisches Signal 114 als gefiltertes Ausgangssignal an, welches gegenüber dem Durchlauf des schnellen Signalpfades 112 unverzögert ist.

In diesem erfindungsgemäßen Verfahren wird im schnellen Signalpfad 112 eine schnelle nichtlineare Filterung vorgenommen, wobei der Störanteil des physiologischen Signals nicht vollständig durch die schnelle Filterung unterdrückt wird. Die schnelle Filterung im schnellen Signalpfad 112 wird dabei vorzugsweise auf nichtlineare Weise durchgeführt.

In diesem erfindungsgemäßen Verfahren wird im langsamen Signalpfad 113 eine langsame Filterung vorgenommen, wobei der Störanteil des physiologischen Signals im Wesentlichen vollständig durch die langsame Filterung unterdrückt wird. Die langsame Filterung im langsamen Signalpfad 113 wird dabei vorzugsweise auf lineare Weise durchgeführt.

In diesem erfindungsgemäßen Verfahren werden der langsame Signalpfad 113 und der schnelle Signalpfad 112 parallel durchlaufen, wobei sich nach dem parallelen Durchlauf ein entstörtes Signal 114 als gefiltertes Ausgangssignal ergibt, welches im Wesentlichen den Nutzanteil des physiologischen Signals enthält und in dem der Störanteil des physiologischen Signals im Wesentlichen unterdrückt wird.

### Bezugszeichenliste

- 100: Filtervorrichtung
- 101: Eingangssignal
- 101a: pneumatische Signale
- 102: schneller Signalpfad
- 103: langsamer Signalpfad
- 104: Ausgangssignal
- 106: Signalleitung
- 107: Artefakt-Analyse-Block
- 110: Filtervorrichtung
- 111: Eingangssignal
- 112: schneller Signalpfad
- 113: langsamer Signalpfad
- 114: Ausgangssignal
- 116: Signalleitung, Verbindung, Parameter
- 117: Signalleitung, Verbindung, Rückführungssignal
- 200: Filtervorrichtung
- 201-1 bis 201-n: Eingangssignal
- 202-1 bis 202-n: schneller Signalpfad
- 203-1 bis 203-n: langsamer Signalpfad
- 204-1 bis 204-n: Ausgangssignal
- 205: Verrechnungs-/Entscheidungseinheit
- 300: Filtervorrichtung
- 301: Eingangssignal
- 302: schneller Signalpfad
- 303: langsamer Signalpfad
- 304: Ausgangssignal
- 306: Signalleitung, Verbindung,
- 307: Signalleitung, Verbindung,
- 308: Signalleitung , Verbindung,
- 309: externes Gerät
- 310: Filtervorrichtung
- 311: Eingangssignal
- 312: schneller Signalpfad
- 313: langsamer Signalpfad
- 314: Ausgangssignal
- 316: Signalleitung, Verbindung,
- 317: Signalleitung, Verbindung,
- 319: externes Gerät
- 400: Filtervorrichtung
- 401-1 bis 401-n: Eingangssignal
- 402-1 bis 402-n: schneller Signalpfad
- 403-1 bis 403-n: langsamer Signalpfad
- 404-1 bis 404-n: Ausgangssignal
- 405: Verrechnungs-/Entscheidungseinheit
- 409-1: externes Gerät
- 600: Filtervorrichtung
- 601: Eingangssignal
- 602: schneller Signalpfad
- 603: langsamer Signalpfad
- 604: Ausgangssignal
- 605: Baselinefilter
- 606: Block zum Entfernen eines EKG-Signals
- 607: Hüllkurvenberechnungseinheit
- 608: Einheit zum Berechnen eines Qualitätsmaßes
- 609: EKG-Signalerkennungseinheit
- 610: Einheit zur Bestimmung der Höhe und des Zeitpunkts des EKGs
- 611: Einheit zur Schwellwertberechnung
- 1001: QRS-Detektor
- 1002: P-Wellen-Detektor
- 1003: Netzartefakt-Detektor
- 1004: Crosstalk-Detektor
- 1005: Schluckauf-Detektor
- 1006: weitere Detektoren
- 1008: Baseline-Filter
- 1009: Artefakt-Entfernung
- 1010: Hüllkurvenberechnung
- 1011: EKG-Parameter
- 1012: Crosstalk-Parameter
- 1013: Schluckaufparameter
- 1014: Netzartefaktparameter
- 1015: Parameter für Spontanatmung

## Patentansprüche

1. Filtervorrichtung (110; 300) zur Verwendung bei der Steuerung eines Beatmungsgeräts mit:
einem Signaleingang (111; 301; 311), an dem ein Eingangssignal anliegt, das einen Nutzanteil und einen Störanteil enthält, wobei das Eingangssignal ein physiologisches Signal von einem Patienten ist;
einem schnellen Signalpfad (112; 302; 312) und einem parallel dazu angeordneten langsamen Signalpfad (113; 303; 313);
wobei der Signaleingang (111; 301; 311) mit Eingängen des schnellen Signalpfades und des langsamen Signalpfades gekoppelt ist, so dass der schnelle Signalpfad und der langsame Signalpfad von dem physiologischen Signal durchlaufen werden;
wobei der schnelle Signalpfad ein Filter enthält, um eine schnelle Filterung des Eingangssignals zu bewirken;
wobei der langsame Signalpfad ein Filter enthält, um eine langsame Filterung des Eingangssignals zu bewirken;
wobei die Signaldurchlaufzeit im schnellen Signalpfad mindestens um den Faktor 2 geringer ist als die Signaldurchlaufzeit im langsamen Signalpfad;
wobei ein Ausgang des langsamen Signalpfades über eine Signalleitung (116; 306; 316) mit dem schnellen Signalpfad gekoppelt ist, um das Verhalten des schnellen Signalpfades zu beeinflussen;
einem Signalausgang (114; 304; 314), der mit einem Ausgang des schnellen Signalpfades gekoppelt ist und an dem ein Ausgangssignal anliegt, das im Wesentlichen die Nutzanteile des Eingangssignals enthält; und
einer weiteren Signalleitung (117; 317), über die ein Signal von dem schnellen Signalpfad an den langsamen Signalpfad zurückgeführt wird, um das funktionale Verhalten des langsamen Signalpfades zu beeinflussen; oder
einer Signalleitung (308), über die ein Ausgang (304) des schnellen Signalpfades (302) mit einem Eingang des langsamen Signalpfades (303) gekoppelt ist, um das funktionale Verhalten des langsamen Signalpfades zu beeinflussen;
wobei das am Signaleingang (111; 301; 311) anliegende physiologische Signal, das nach seiner Filterung durch die Filtervorrichtung am Signalausgang anliegt, zur Ansteuerung eines Beatmungsgerätes (309; 319) verwendet wird; und
wobei der schnelle Signalpfad ein Filter mit nichtlinearen Eigenschaften aufweist und der langsame Signalpfad ein Filter mit linearen Eigenschaften oder ein adaptives Filter aufweist.

2. Filtervorrichtung nach Anspruch 1, bei der der langsame Signalpfad (103; 113; 203-1 bis 203-n; 303; 313; 603) mit einem Artefakt-Analyse-Block (107) versehen ist, der Eingänge für Zusatzsignale aufweist.

3. Filtervorrichtung (200; 400) zur Verwendung bei der Steuerung eines Beatmungsgeräts mit:
einer Mehrzahl von Signaleingängen (201-1 bis 201-n; 401-1 bis 401-n), an denen jeweils ein Eingangssignal anliegt, das einen Nutzanteil und einen Störanteil enthält, wobei die Eingangssignale physiologische Signale von einem Patienten sind;
einer Mehrzahl von parallel angeordneten schnellen Signalpfaden (202-1 bis 202-n; 402-1 bis 402-n);
einer Mehrzahl von parallel angeordneten langsamen Signalpfaden (203-1 bis 203-n; 403-1 bis 403-n);
wobei jeder der Mehrzahl von Signaleingängen jeweils mit einem Eingang der Mehrzahl von parallel angeordneten schnellen Signalpfaden und der Mehrzahl von parallel angeordneten langsamen Signalpfaden gekoppelt ist;
wobei jeder schnelle Signalpfad (202-1 bis 202-n; 402-1 bis 402-n) ein Filter enthält, um eine schnelle Filterung des Eingangssignals zu bewirken;
wobei jeder langsame Signalpfad (203-1 bis 203-n; 403-1 bis 403-n) ein Filter enthält, um eine langsame Filterung des Eingangssignals zu bewirken;
wobei die Signaldurchlaufzeit im schnellen Signalpfad mindestens um den Faktor 2 geringer ist als die Signaldurchlaufzeit im langsamen Signalpfad;
wobei die Ausgänge von jedem langsamen Signalpfad (203-1 bis 203-n; 403-1 bis 403-n) über jeweilige Signalleitungen mit zugehörigen Eingängen einer Verrechnungs-/Entscheidungseinheit (205; 405) gekoppelt sind;
wobei jeder schnelle Signalpfad (202-1 bis 202-n; 402-1 bis 402-n) über jeweilige Signalleitungen mit zugehörigen Ausgängen der Verrechnungs-/ Entscheidungseinheit (205; 405) gekoppelt ist, um dadurch die Ergebnisse der langsamen Filterung eines Signals beliebig auf die schnelle Filterung eines anderen Signals anwenden zu können; und
einer Mehrzahl von Signalausgängen (204-1 bis 204-n; 404-1 bis 404-n), die jeweils mit Ausgängen der schnellen Signalpfade (202-1 bis 202-n; 402-1 bis 402-n) gekoppelt sind und an denen jeweils Ausgangssignale anliegen, die im Wesentlichen Nutzanteile der Eingangssignale enthalten;
wobei die an den Signaleingängen anliegenden physiologischen Signale, die nach ihrer Filterung durch die Filtervorrichtung an den Signalausgängen anliegen, zur Ansteuerung eines Beatmungsgerätes verwendet werden;
wobei der jeder der Mehrzahl von schnellen Signalpfaden ein Filter mit nichtlinearen Eigenschaften aufweist und jeder der Mehrzahl von langsamen Signalpfaden ein Filter mit linearen Eigenschaften oder ein adaptives Filter aufweist; und
wobei die Ausgänge (404-1 bis 404-n) der schnellen Signalpfade (402-1 bis 402-n) über jeweilige Signalleitungen mit zugehörigen Eingängen der Verrechnungs-/Entscheidungseinheit (405) gekoppelt sind.

4. Filtervorrichtung nach Anspruch 3, bei der der langsame Signalpfad (113; 203-1 bis 203-n; 303; 313; 603) mit einem Artefakt-Analyse-Block (107) versehen ist, der Eingänge für Zusatzsignale aufweist.

5. Verfahren zur Echtzeitfilterung eines physiologischen Signals von einem Patienten, das einen Nutzanteil und einen Störanteil enthält, zur Verwendung bei der Steuerung eines Beatmungsgeräts, umfassend:
Einspeisen des physiologischen Signals in einen schnellen Signalpfad (112; 302; 312) mit einem Signaleingang und einem Signalausgang und in einen parallel dazu angeordneten langsamen Signalpfad (113; 303; 313) mit einem Signaleingang und einem Signalausgang, wobei der schnelle Signalpfad und der langsame Signalpfad von dem physiologischen Signal durchlaufen werden,
Einspeisen eines Parameters über eine Signalleitung (116; 306; 316), welche einen Signalausgang des langsamen Signalpfades (113; 303; 313) mit dem schnellen Signalpfad (112; 302; 312) verbindet, um das Verhalten des schnellen Signalpfades zu beeinflussen, und
Zurückführen eines Rückführungssignals über eine weitere Signalleitung (117; 317) von dem schnellen Signalpfad (112; 302; 312) an den langsamen Signalpfad (113; 303; 313), um das funktionale Verhalten des langsamen Signalpfades zu beeinflussen,
wobei das physiologische Signal parallel in den Eingang des schnellen Signalpfades (112; 302; 312) und in den Eingang des langsamen Signalpfades (113; 303; 313) eingekoppelt wird,
wobei der schnelle Signalpfad (112; 302; 312) schnell durchlaufen wird und am Signalausgang des schnellen Signalpfades (112; 302; 312) ein gefiltertes Ausgangssignal anliegt, das im Wesentlichen den Nutzanteil des physiologischen Signals enthält und in dem der Störanteil des physiologischen Signals nicht vollständig durch die schnelle Filterung unterdrückt ist,
wobei der langsame Signalpfad (113; 303; 313) langsam durchlaufen wird und am Signalausgang des langsamen Signalpfades (113; 303; 313) ein gefiltertes Ausgangssignal anliegt, das im Wesentlichen den Nutzanteil des physiologischen Signals enthält und in dem der Störanteil des physiologischen Signals im Wesentlichen durch die langsame Filterung unterdrückt ist,
wobei am Signalausgang (114; 304; 314) unverzögert gegenüber dem Signalausgang des schnellen Signalpfades (112; 302; 312) ein gefiltertes Ausgangssignal anliegt, das im Wesentlichen den Nutzanteil des physiologischen Signals enthält und in dem der Störanteil des physiologischen Signals im Wesentlichen unterdrückt ist, und
wobei die Signaldurchlaufzeit im schnellen Signalpfad mindestens um den Faktor 2 geringer ist als die Signaldurchlaufzeit im langsamen Signalpfad.

6. Verfahren nach Anspruch 5, wobei im schnellen Signalpfad (112; 302; 312) der Störanteil des physiologischen Signals nicht vollständig unterdrückt wird.

7. Verfahren nach Anspruch 5, wobei im langsamen Signalpfad (113; 303; 313) der Störanteil des physiologischen Signals im Wesentlichen vollständig unterdrückt wird.

## Claims

1. A filter apparatus (110; 300) for use in the control of a respirator comprising:
a signal input (111; 301; 311) at which an input signal is present, which input signal contains a useful component and a noise component, wherein the input signal is a physiological signal from a patient;
a fast signal path (112; 302; 312) and a slow signal path (113; 303; 313) arranged in parallel to the fast signal path;
wherein the signal input (111; 301; 311) is coupled with the inputs of the fast signal path and of the slow signal path, such that the physiological signal passes through the fast signal path and through the slow signal path;
wherein the fast signal path contains a filter to bring about fast filtering of the input signal;
wherein the slow signal path contains a filter to bring about slow filtering of the input signal;
wherein the signal travel time in the fast signal path is shorter by a factor of at least 2 than the signal travel time in the slow path;
wherein an output of the slow signal path is coupled with the fast signal path via a signal line (116; 306; 316) to affect the characteristic of the fast signal path;
a signal output (114; 304; 314) coupled with an output of the fast signal path and at which an output signal, which contains essentially useful components of the input signal, is present; and
another signal line (117; 317) via which a signal is returned from the fast signal path to the slow signal path to affect the functional characteristic of the slow filter path; or
a signal line (308) via which an output (304) of the fast signal path (302) is coupled with an input of the slow signal path (303) to affect the functional characteristic of the slow filter path;
wherein the physiological signal, which is present at the signal input (111; 301; 311) and which is present at the signal output after its filtering by the filter apparatus, is used to drive a respirator (309; 319); and
wherein the fast signal path has a filter with nonlinear properties and the slow signal path has a filter with linear properties or an adaptive filter.

2. A filter apparatus in accordance with claim 1, wherein the slow signal path (103; 113; 203-1 to 203-n; 303; 313; 603) is provided with an artifact analysis block (107), which has inputs for additional signals.

3. A filter apparatus (200; 400) for use in the control of a respirator comprising:
a plurality of signal inputs (201-1 to 201-n; 401-1 to 401-n) at each of which an input signal is present, which input signal contains a useful component and a noise component, wherein the input signals are physiological signals from a patient;
a plurality of fast signal paths (202-1 to 202-n; 402-1 to 402-n) arranged in parallel;
a plurality of slow signal paths (203-1 to 203-n; 403-1 to 403-n) arranged in parallel;
wherein each of the plurality of signal inputs is coupled with an input of the plurality of fast signal paths arranged in parallel and of the plurality of slow signal paths arranged in parallel;
wherein each fast signal path (202-1 to 202-n; 402-1 to 402-n) contains a filter to bring about fast filtering of the input signal;
wherein each slow signal path (203-1 to 203-n; 403-1 to 403-n) contains a filter to bring about slow filtering of the input signal;
wherein the signal travel time in the fast signal path is shorter by a factor of at least 2 than the signal travel time in the slow path;
wherein the outputs of each slow signal path (203-1 to 203-n; 403-1 to 403-n) are coupled with corresponding inputs of an accounting/decision unit (205; 405) via respective signal lines;
wherein each fast signal path (202-1 to 202-n; 402-1 to 402-n) is coupled with corresponding outputs of the accounting/decision unit (205; 405) via respective signal lines to thereby apply the results of the slow filtering of a signal to the fast filtering of another signal; and
a plurality of signal outputs (204-1 to 204-n; 404-1 to 404-n) which are each coupled with outputs of the fast signal paths (202-1 to 202-n; 402-1 to 402-n) and at which respective output signals, which contain essentially useful components of the input signals, are present;
wherein the physiological signals, which are present at the signal inputs and which are present at the signal outputs after their filtering by the filter apparatus, are used to drive a respirator;
wherein each of the plurality of fast signal paths has a filter with nonlinear properties and each of the plurality of slow signal paths has a filter with linear properties or an adaptive filter; and
wherein the outputs (404-1 to 404-n) of the fast signal paths (402-1 to 402-n) are coupled with corresponding inputs of the accounting/decision unit (405) via respective signal lines.

4. A filter apparatus in accordance with claim 3, wherein the slow signal path (113; 203-1 to 203-n; 303; 313; 603) is provided with an artifact analysis block (107), which has inputs for additional signals.

5. A method for real-time filtering of a physiological signal from a patient, which signal contains a useful component and a noise component, for use in the control of a respirator, the method comprising the steps of:
applying the physiological signal to a fast signal path (112; 302; 312) having a signal input and a signal output, and to a slow signal path (113; 303; 313) arranged in parallel to the fast signal path and having a signal input and a signal output, wherein the physiological signal passes through the fast signal path and through the slow signal path,
applying a parameter via a signal line (116; 306; 316) which connects a signal output of the slow signal path (113; 303; 313) with the fast signal path (112; 302; 312) to affect the characteristic of the fast signal path, and
returning a feedback signal via another signal line (117; 317) from the fast signal path (112; 302; 312) to the slow signal path (113; 303; 313) to affect the functional characteristic of the slow filter path,
wherein the physiological signal is coupled in parallel into the input of the fast signal path (112; 302; 312) and into the input of the slow signal path (113; 303; 313),
wherein the fast signal path (112; 302; 312) is quickly passed, and wherein a filtered output signal is present at the signal output of the fast signal path (112; 302; 312), which output signal contains essentially the useful component of the physiological signal and in which output signal the noise component of the physiological signal is not suppressed completely by the fast filtering,
wherein the slow signal path (113; 303; 313) is slowly passed, and wherein a filtered output signal is present at the signal output of the slow signal path (113; 303; 313), which output signal contains essentially the useful component of the physiological signal and in which output signal the noise component of the physiological signal is essentially suppressed by the slow filtering,
wherein a filtered output signal, which contains essentially the useful component of the physiological signal and in which the noise component of the physiological signal is essentially suppressed, is present at the signal output (114; 304; 314) without time delay relative to the signal output of the fast signal path (112; 302; 312), and
wherein the signal travel time in the fast signal path is shorter by a factor of at least 2 than the signal travel time in the slow path.

6. A method in accordance with claim 5, wherein the noise component of the physiological signal is not suppressed completely in the fast signal path (112; 302; 312).

7. A method in accordance with claim 5, wherein the noise component of the physiological signal is suppressed essentially completely in the slow signal path (113; 303; 313).

## Revendications

1. Dispositif de filtrage (110; 300) destiné à une utilisation pour la commande d'un respirateur artificiel, comprenant :
une entrée de signal (111; 301; 311), à laquelle est appliqué un signal d'entrée qui comprend une composante utile et une composante parasite, le signal d'entrée étant un signal physiologique d'un patient;
un chemin de signal rapide (112; 302; 312) et un chemin de signal lent (113; 303; 313) disposé parallèlement à celui-ci;
où l'entrée de signal (111; 301; 311) est couplée à des entrées du chemin de signal rapide et du chemin de signal lent, de sorte que le chemin de signal rapide et le chemin de signal lent sont parcourus par le signal physiologique;
où le chemin de signal rapide contient un filtre destiné à opérer un filtrage rapide du signal d'entrée;
où le chemin de signal lent contient un filtre destiné à opérer un filtrage lent du signal d'entrée;
où le temps de passage de signal dans le chemin de signal rapide est inférieur d'au moins un facteur 2 au temps de passage de signal dans le chemin de signal lent;
où une sortie du chemin de signal lent est couplée via une ligne de transmission de signaux (116; 306; 316) au chemin de signal rapide pour agir sur le comportement du chemin de signal rapide;
une sortie de signal (114; 304, 314) qui est couplée à une sortie du chemin de signal rapide et à laquelle est appliqué un signal de sortie qui comporte essentiellement les composantes utiles du signal d'entrée; et
une autre ligne de transmission de signaux (117; 317), par l'intermédiaire de laquelle un signal est renvoyé du chemin de signal rapide au chemin de signal lent pour agir sur le comportement fonctionnel du chemin de signal lent; ou
une ligne de transmission de signaux (308), par l'intermédiaire de laquelle une sortie (304) du chemin de signal rapide (302) est couplée à une entrée du chemin de signal lent (303) pour agir sur le comportement fonctionnel du chemin de signal lent;
où le signal physiologique qui est appliqué à l'entrée de signal (111; 301; 311) et qui, après son filtrage par le dispositif de filtrage, est appliqué à la sortie de signal, est utilisé pour l'activation d'un respirateur artificiel (309; 319); et
où le chemin de signal rapide présente un filtre avec des caractéristiques non linéaires, et le chemin de signal lent présente un filtre avec des caractéristiques linéaires, ou un filtre adaptatif.

2. Dispositif de filtrage selon la revendication 1, dans lequel le chemin de signal lent (103; 113; 203-1 à 203-n; 303; 313; 603) est doté d'un bloc d'analyse d'artéfact (107) qui présente des entrées pour des signaux supplémentaires.

3. Dispositif de filtrage (200, 400) destiné à une utilisation pour la commande d'un respirateur artificiel, comprenant :
une pluralité d'entrées de signal (201-1 à 201-n; 401-1 à 401-n), auxquelles est appliqué respectivement un signal d'entrée qui comprend une composante utile et une composante parasite, les signaux d'entrée étant des signaux physiologiques d'un patient;
une pluralité de chemins de signal rapides (202-1 à 202-n; 402-1 à 402-n) parallèles;
une pluralité de chemins de signal lents (203-1 à 203-n; 403-1 à 403-n) parallèles;
où chacune de la pluralité d'entrées de signal est couplée respectivement à une entrée de la pluralité de chemins de signal rapides parallèles et de la pluralité de chemins de signal lents parallèles;
où chaque chemin de signal rapide (202-1 à 202-n; 402-1 à 402-n) contient un filtre destiné à opérer un filtrage rapide du signal d'entrée;
où chaque chemin de signal lent (203-1 à 203-n; 403-1 à 403-n) contient un filtre destiné à opérer un filtrage lent du signal d'entrée;
où le temps de passage de signal dans le chemin de signal rapide est inférieur d'au moins un facteur 2 au temps de passage de signal dans le chemin de signal lent;
où les sorties de chaque chemin de signal lent (203-1 à 203-n; 403-1 à 403-n) sont couplées via des lignes de transmission de signaux respectives à des entrées associées d'une unité de calcul/décision (205; 405);
où chaque chemin de signal rapide (202-1 à 202-n; 402-1 à 402-n) est couplé via des lignes de transmission de signaux respectives à des sorties associées de l'unité de calcul/décision (205; 405), afin de pouvoir appliquer ainsi librement les résultats du filtrage lent d'un signal au filtrage rapide d'un autre signal; et
une pluralité de sorties de signal (204-1 à 204-n; 404-1 à 404-n) qui sont couplées respectivement à des sorties des chemins de signal rapides (202-1 à 202-n; 402-1 à 402-n) et auxquelles sont appliqués respectivement des signaux de sortie qui comportent essentiellement des composantes utiles des signaux d'entrée;
où les signaux physiologiques qui sont appliqués aux entrées de signal et qui, après leur filtrage par le dispositif de filtrage, sont appliqués aux sorties de signal, sont utilisés pour l'activation d'un respirateur artificiel;
où chacun de la pluralité de chemins de signal rapides présente un filtre avec des propriétés non linéaires, et chacun de la pluralité de chemins de signal lents présente un filtre avec des caractéristiques linéaires, ou un filtre adaptatif; et
où les sorties (404-1 à 404-n) des chemins de signal rapides (402-1 à 402-n) sont couplées via des lignes de transmission de signaux respectives à des entrées associées de l'unité de calcul/décision (405).

4. Dispositif de filtrage selon la revendication 3, dans lequel le chemin de signal lent (113; 203-1 à 203-n; 303; 313; 603)) est doté d'un bloc d'analyse d'artéfact (107) qui présente des entrées pour des signaux supplémentaires.

5. Procédé de filtrage en temps réel d'un signal physiologique d'un patient, qui présente une composante utile et une composante parasite, destiné à une utilisation pour la commande d'un respirateur artificiel, comprenant :
l'injection du signal physiologique dans un chemin de signal rapide (112; 302; 312) comprenant une entrée de signal et une sortie de signal, et dans un chemin de signal lent (113; 303; 313) parallèle à celui-ci et comprenant une entrée de signal et une sortie de signal, le chemin de signal rapide et le chemin de signal lent étant parcourus par le signal physiologique,
injection d'un paramètre via une ligne de transmission de signaux (116; 306; 316) qui relie une sortie de signal du chemin de signal lent (113; 303; 313) au chemin de signal rapide (112; 302; 312) pour agir sur le comportement du chemin de signal rapide, et
renvoi d'un signal de retour, via une autre ligne de transmission de signaux (117; 317), du chemin de signal rapide (112; 302; 312) au chemin de signal lent (113; 303; 313), pour agir sur le comportement fonctionnel du chemin de signal lent,
où le signal physiologique est couplé parallèlement dans l'entrée du chemin de signal rapide (112; 302; 312) et dans l'entrée du chemin de signal lent (113; 303; 313);
où le chemin de signal rapide (112; 302; 312) est parcouru rapidement et un signal de sortie filtré est appliqué à la sortie de signal du chemin de signal rapide (112; 302; 312), signal qui comporte essentiellement la composante utile du signal physiologique et dans lequel la composante parasite du signal physiologique n'est pas supprimée complètement par le filtrage rapide,
où le chemin de signal lent (113; 303; 313) est parcouru lentement et un signal de sortie filtré est appliqué à la sortie de signal du chemin de signal lent (113; 303; 313), signal qui comporte essentiellement la composante utile du signal physiologique et dans lequel la composante parasite du signal physiologique est sensiblement supprimée par le filtrage lent,
où un signal de sortie filtré est appliqué à la sortie de signal (114; 304; 314) sans retard par rapport à la sortie de signal du chemin de signal rapide (112; 302; 312), signal qui comporte essentiellement la composante utile du signal physiologique et dans lequel la composante parasite du signal physiologique est sensiblement supprimée, et
où le temps de passage de signal dans le chemin de signal rapide est inférieur d'au moins un facteur 2 au temps de passage de signal dans le chemin de signal lent.

6. Procédé selon la revendication 5, selon lequel, dans le chemin de signal rapide (112; 302; 312), la composante parasite du signal physiologique n'est pas complètement supprimée.

7. Procédé selon la revendication 5, selon lequel, dans le chemin de signal lent (113; 303; 313), la composante parasite du signal physiologique est sensiblement supprimée.
